(19) Europäisches Patentamt
European Patent Office
Office européen des brevets

(11) **EP 3 848 018 A1**

(12) # EUROPEAN PATENT APPLICATION

(43) Date of publication:
**14.07.2021 Bulletin 2021/28**

(21) Application number: **20150812.4**

(22) Date of filing: **08.01.2020**

(51) Int Cl.:
*A61K 8/60* (2006.01)          *A61K 8/73* (2006.01)
*A61K 8/97* (2017.01)          *A61K 8/9717* (2017.01)
*A61K 8/9789* (2017.01)          *A61Q 5/02* (2006.01)
*A61Q 5/12* (2006.01)          *A61Q 17/00* (2006.01)
*A61Q 19/00* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME
KH MA MD TN**

(71) Applicant: **CreaSearch BV
3300 Tienen (BE)**

(72) Inventor: **BOOTEN, Karl
B-3300 Tienen (BE)**

(74) Representative: **IPLodge bv
Technologielaan 9
3001 Heverlee (BE)**

(54) **COSMETIC COMPOSION COMPRISING A SAPONIN AND A SACCHARIDE POLYMER**

(57)     The invention pertains to a cosmetic composition comprising: at least one saccharide polymer selected from the group consisting of starch hydrolysates, fructans, fructooligosaccharides, and mixtures thereof; and at least one saponin. The invention further relates to the use of the cosmetic composition in the preparation of a rinse-off cosmetic product or a leave-on cosmetic product.

**EP 3 848 018 A1**

## Description

### Field of the Invention

The present invention relates to cosmetic compositions and to the use of such compositions in the preparation of a cosmetic product.

### Background

In the past years, public concern about the safety of synthetic compounds used in cosmetic products, especially in view of their accumulation in the body and subsequent health effect, have motivated health authorities to reduce the maximum allowable concentrations of some compounds in cosmetic products or even ban such compounds. Furthermore, a trend in the global cosmetics market has recently emerged to replace some polemic ingredients, such as components derived from petroleum or synthetic surfactants which are perceived as being aggressive or even toxic to the human body, with less controversial alternatives, preferably from a natural source. Due to the importance of the continuously expanding global cosmetics products market, which is expected to reach more than 800 billion USD by 2023, such trends are having a profound socioeconomic and ecological impact.

While it is the wish of the customer to see these ingredients replaced with alternatives from a less controversial or preferably a natural source, it has also been found that he or she does not tolerate that such adjustments affect the cosmetic properties of the new product in a negative way. Not only does an alternative composition have to dispose of e.g. the same cleansing capabilities, the product user also needs to have a similar or even better "experience" with the product. The product experience is thereby often expressed in appreciations of a personal nature, such as e.g. the pleasant "feeling" the rinse-off shower gel product leaves on the skin after washing, or the ease with which dried hair can be combed after being washed with a shampoo. It is therefore crucial that a complete or partial replacement of unwanted ingredients by alternatives does not harm the consumer experience.

There is therefore a need for cosmetic compositions which allow for the complete or partial replacement of ingredients, which may be of a synthetic origin or which may be perceived as aggressive towards skin and/or hair, with alternative compounds, preferably of a natural origin, while providing a product with similar, preferably improved, product cosmetic properties that offers the product user at least a similar or an improved product experience.

International patent application publication no. WO 93/00067 A1, in the name of BIOEUROPE, discloses cosmetic compositions providing a suitable medium for the development of beneficial endogenous flora, and including at least one oligosaccharide selected from the group consisting of gluco-oligosaccharides, fructo-oligosaccharides, $\alpha$- and $\beta$-galacto-oligosaccharides and mixtures thereof. Examples of compositions are liquid soap, shampoo, body lotion, face cream and vaginal gel. That application does not disclose compositions comprising a saponin.

European patent application publication no. EP 1 380 284 A1, in the name of L'ORÉAL, discloses a detergent and conditioning composition comprising one or more anionic surfactants (a), one or more amphoteric, cationic and/or non-ionic surfactants (b) with a ratio by weight (a)/(b) of 1 or more and a polysaccharide which is a non-ionic or anionic fructan or starch hydrolysate with a dextrose equivalent below 20.

### Summary of the Invention

According to an aspect of the present invention, there is provided a cosmetic composition comprising: at least one saccharide polymer selected from the group consisting of starch hydrolysates, fructans, fructooligosaccharides, and mixtures thereof; and at least one saponin.

The term "saccharide polymer" is used herein to denote oligosaccharides and polysaccharides. "Oligosaccharides" are saccharide polymers containing a small number (up to ten) of monosaccharides (simple sugars). In particular, fructooligosaccharides (FOS) are short chains of fructose units. "Polysaccharides" are saccharide polymers containing a larger number (more than ten) of monosaccharides (simple sugars). In particular, fructans are long chains of fructose units. The term fructan will be understood to include inulin, which are composed mainly of fructose units, and typically have a terminal glucose.

Both saponins and saccharide polymers as used in the invention are natural products. They therefore do not suffer negative consumer perception as described above.

It is an advantage of embodiments of the present invention that the combination of said at least one saponin and said at least one saccharide polymer as described herein results in cosmetic compositions which may be a cosmetic product or which may be part of a cosmetic product, wherein said cosmetic composition or cosmetic product as described hereabove has improved cosmetic properties in comparison with similar compositions missing at least one of the components described hereabove. The nature of the cosmetic parameter which is improved by said combination may in general be dependent on the type of cosmetic product.

In an embodiment, the cosmetic composition according to the present invention may be a shampoo composition (i.e., a composition for the treatment and/or cleansing of hair). It has been found that shampoo compositions according to the invention, advantageously present improved wet combing, wet detangling, and dry combing (after treating the hair with the shampoo composition and subsequent drying of the hair) properties in comparison with similar compositions that contained none or only one of said two components.

In an embodiment, the cosmetic composition according to the present invention may be a shower gel composition (i.e, a composition for cleansing the skin, typically used under the shower). It has been found that shower gel compositions according to the invention, advantageously present an improved "feeling" on the skin, when evaluated one minute or thirty minutes after the application and rinsing off of the shower gel composition.

In an embodiment, the cosmetic composition according to the present invention may be a leave-on composition (e.g., a skin cream or lotion). It has been found that leave-on compositions according to the invention advantageously present an improved afterfeel. The compositions according to the invention have been found to have a "richer" feeling without feeling "greasy". This is an unexpected surprising effect, since normally compositions that are intended to give said rich feeling also give a greasier feeling, which is considered undesirable.

In an embodiment of the cosmetic composition according to the present invention, the at least one saccharide polymer comprises a levan-type polymer or an inulin-type polymer.

Levan-type polymers are composed of fructooligosaccharide of polyfructose molecules wherein the fructosyl units are mostly connected to each other by β-2,6-linkages. Levan is naturally synthesized by certain plant species and by certain bacteria.

Inulin-type polymers are composed of fructooligosaccharide or polyfructose molecules of which the fructosyl units are exclusively or mainly connected to each other by β-2,1-linkages and which furthermore may or may not bear one terminal glucose unit. The term "inulin-type polymer", as used herein, refers both to inulin and oligofructose. For the sake of completeness, it is noted that the term "inulin-type fructan" is sometimes used in literature to refer to the notion of "inulin-type polymer" as defined above. Inulin-type polymer is naturally synthesized by many plant species but can also originate from bacterial activity.

In an embodiment of the cosmetic composition according to the present invention, said at least one saccharide polymer is present in the composition in a concentration of at least 0.01 wt% to at most 8 wt%, by weight relative to the total weight of the composition.

It is an advantage of said embodiment that providing said at least one saccharide polymer in this concentration allows for the advantageous cosmetic properties described herein.

In an embodiment of the cosmetic composition according to the present invention, said at least one saponin is present in the composition in a concentration of at least 0.001 wt% to at most 15 wt% by weight relative to the total weight of the composition.

It is an advantage of said embodiment that providing said at least one saponin in this concentration allows for the advantageous cosmetic properties described herein.

In an embodiment, the cosmetic composition according to the present invention further comprises at least one of the following: an anionic surfactant, a cationic surfactant, an amphoteric surfactant, a non-ionic surfactant, wherein said non-ionic surfactant is exclusive of a saponin.

In an embodiment, the cosmetic composition according to the present invention further comprises at least one oil selected from jojoba oil, isoamyl laurate, and cyclopentasiloxane; said at least one oil being comprised in a hydrophobic phase of a stable emulsion.

The term "emulsion" in the context of the present invention refers to a system whereby at least two non-miscible liquids are mixed together wherein one phase, the dispersed phase, gets dispersed in the other phase, the continuous phase. This typically involves mixing a phase component of hydrophobic nature, forming the hydrophobic phase in the envisaged emulsion, with a second phase component of hydrophilic nature, forming the hydrophilic phase in the envisaged emulsion. The "stability" of the emulsion refers to the ability of an emulsion to resist change in its properties over time, as will be explained in more detail hereinbelow.

It is an advantage of this embodiment that it allows the composition to remain in a stable emulsion form. This type of composition is particularly suitable for use as a leave-on product (e.g., a skin cream or lotion) or very specific types of rinse-off products (e.g. hair conditioner).

In an embodiment, the cosmetic composition according to the present invention further comprises at least one emulsion breaking agent selected from the following:

- an antimicrobial agent, such as a medium-chain length linear vicinal diol, glyceryl caprylate, or caprylyl glycol;
- an electrolyte, such as $MgSO_4$ or NaCl;
- a cationic molecule, such as cationic surfactants such as cetrimonium chloride;
- an oxidizing agent, such as hydrogen peroxide in a concentration between 10 and 30% (v/v);
- a sunless tanning agent, such as dihydroxy acetone (DHA);

- a reducing agent, such as potassium thioglycolate;
- an alpha hydroxy acid, such as glycolic acid;
- an alpha and beta hydroxy acid, such as salicylic acid;
- a surfactant, such as cocamidopropyl betaine, sodium cocoyl glutamate, or sodium lauroyl glutamate;
- a $C_1$-$C_7$ alcohol, such as ethanol;
- a perfume compound.

The inventor has found that the combination of the at least one saponin and the at least one saccharide polymer according to the invention may result in obtaining a stable emulsion, even when adding amounts of emulsion breakers. Accordingly, a wide variety of emulsion-based products, in particular leave-on products, can be produced on the basis of the cosmetic composition of the present invention.

In an embodiment, the cosmetic composition according to the present invention is a leave-on cosmetic product or is part of a leave-on cosmetic product, and said at least one saccharide polymer is present in the cosmetic product in a concentration of at least 0.01 wt% to at most 8 wt%, by weight relative to the total weight of the cosmetic product.

It is an advantage of this embodiment that providing said at least one saccharide polymer in this concentration allows for the advantageous cosmetic properties described herein for leave-on cosmetic products.

In a particular embodiment, the at least one saponin is present in the cosmetic product in a concentration of at least 0.001 wt% to at most 5 wt% by weight relative to the total weight of the cosmetic product.

It is an advantage of this embodiment that providing said at least one saponin in this concentration allows for the advantageous cosmetic properties described herein for leave-on cosmetic products.

In a particular embodiment, the weight ratio of said at least one saponin to the total of all surfactants in the composition, excluding said at least one saponin, is at least 1:100 and at most 100:1.

It is an advantage of this embodiment that said weight ratio allows for the advantageous cosmetic properties described herein.

In an embodiment, the cosmetic composition according to the present invention is a rinse-off cosmetic product or is part of a rinse-off cosmetic product, and said at least one saccharide polymer is present in the cosmetic product in a proportion of at least 0.01 wt% to at most 8 wt%, by weight relative to the total weight of the cosmetic product

It is an advantage of this embodiment that providing said at least one saccharide polymer in this concentration allows for the advantageous cosmetic properties described herein for rinse-off cosmetic products.

In a particular embodiment, the at least one saponin is present in the cosmetic product in a concentration of at least 0.1 wt% to at most 15 wt% by weight relative to the total weight of the cosmetic product.

It is an advantage of this embodiment that providing said at least one saponin in this concentration allows for the advantageous cosmetic properties described herein for rinse-off cosmetic products.

It shall be noted that the lower end of this range is higher than the lower end of the range presented above for leave-on products, and that the higher end of this range is likewise higher than the higher end of the range presented above for leave-on products. These higher concentrations are justified in view of the fact that rinse-off products will be in contact with the skin for much shorter durations than leave-on products. In rinse-off products, the saponin will contribute to the detergent or cleansing function of the product, along with the other surfactants present in the product.

In a particular embodiment, the weight ratio of said at least one saponin to the total of all surfactants in the composition, excluding said at least one saponin, is at least 1:100 and at most 100:1.

It is an advantage of this embodiment that said weight ratio allows for the advantageous cosmetic properties described herein.

According to an aspect of the present invention, there is provided a use of the cosmetic composition as described above in the preparation of a rinse-off cosmetic product or a leave-on cosmetic product.

According to an aspect of the present invention, there is provided a process for producing the cosmetic composition as described above, the process comprising extracting saponin from a source using a mixture of water and a water-soluble alcohol; and combining said extracted saponin with at least one saccharide polymer selected from the group consisting of starch hydrolysates, fructans, fructooligosaccharides, and mixtures thereof.

In a particular embodiment, said extraction is performed by use of a mixture of a water-soluble alcohol, preferably ethanol, and water, wherein the alcohol:water ratio is between 60:40 and 80:20.

It is an advantage of this embodiment that said ratio allows reducing the color intensity of the extracted saponin.

**Detailed Description of Embodiments**

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification do not necessarily refer to the same embodiment, although this may be the case. Furthermore, the particular features, structures or characteristics may be combined in any suitable manner, as would be apparent to one of ordinary skill in the art from this disclosure, in one or more embodiments.

Similarly it should be appreciated that in the description of exemplary embodiments of the invention, various features of the invention are sometimes grouped together in a single embodiment, figure, or description thereof for the purpose of streamlining the disclosure and aiding in the understanding of one or more of the various inventive aspects. This method of disclosure, however, is not to be interpreted as reflecting an intention that the claimed invention requires more features than are expressly recited in each claim. Rather, as the following claims reflect, inventive aspects lie in less than all features of a single foregoing disclosed embodiment. Thus, the claims following the detailed description are hereby expressly incorporated into this detailed description, with each claim standing on its own as a separate embodiment of this invention.

Furthermore, while some embodiments described herein include some but not other features included in other embodiments, combinations of features of different embodiments are meant to be within the scope of the invention, and form different embodiments, as would be understood by those in the art. For example, in the following claims, any of the claimed embodiments can be used in any combination.

In the description provided herein, numerous specific details are set forth. However, it is understood that embodiments of the invention may be practiced without these specific details. In other instances, well-known methods, structures and techniques have not been shown in detail in order not to obscure an understanding of this description.

An aspect according to the invention relates to a cosmetic composition comprising at least one saccharide polymer selected from the group consisting of starch hydrolysates, fructans, fructooligosaccharides, and mixtures thereof; and at least one saponin.

According to embodiments of the invention, said cosmetic composition may be a cosmetic product, may be part of a cosmetic product or may be used in a cosmetic product. More in particular, said cosmetic composition is configured to be applied on parts of the human body, including the skin, hair, and mucous membranes.

A cosmetic product can refer herein to a leave-on cosmetic product, as well as a rinse-off cosmetic product. For the purpose of the invention, the term "rinse-off" refers herein to a cosmetic product which is used to be applied to the skin, the hair or mucous membranes, for example to clean them, and which is furthermore intended to be removed after application thereon. For the purpose of the invention, the term "leave-on" refers herein to a cosmetic product which is intended to stay in prolonged contact with the skin, the hair or mucous membranes. According to an aspect of the invention, there is provided a cosmetic product comprising the cosmetic composition described above. Said cosmetic product may be a rinse-off cosmetic product or a leave-on cosmetic product. It will be appreciated by the skilled in the art that the concentration for said at least one saccharide polymer which will be typically applied in the final product is dependent on the type of cosmetic product.

When used as a leave-on product, the cosmetic compositions as described herein will typically form an emulsion, whereby the saponin acts as an emulsifier or a co-emulsifier. It was found that the combination of the at least one saponin and the at least one saccharide polymer according to the invention may result in obtaining a stable emulsion, even when adding amounts further components that tend to act as emulsion breakers, as described herein.

When used as a rinse-off product, the cosmetic compositions as described herein will typically form a gel or a solution, although emulsions may be used for some specific applications. The hair and skin of mammals are prevented from becoming overly dry by an oily or waxy matter, called sebum, that is secreted by sebaceous glands. Over time, the accumulating sebum attracts dirt; the primary purpose of shampoo or soap is to remove the dirty sebum from the hair and/or skin. This is effected by surfactants present in the shampoo or soap. It is known in the state of the art to provide cosmetic compositions with several surfactants. As rinse-off compositions need to clean the skin and/or hair in a short period of time before they are washed off, such compositions typically contain at least one of anionic surfactants, amphoteric surfactants, cationic surfactants and non-ionic surfactants. Such surfactants typically allow for an acceptable cleansing effect which needs to be achieved during the relatively short period of time of a bath or a shower. For typical compositions, anionic surfactants make up the bulk of the surfactant ingredients. However, anionic surfactants also seem to have the highest effect on lipids and proteins which are naturally present on the skin and the hair, by e.g. denaturation of the latter. Anionic surfactants are therefore considered as a main source for damaging of such components.

It is known in the state of the art to provide cosmetic compositions with several surfactants. As rinse-off compositions need to clean the skin and/or hair in a short period of time and are then washed off, such compositions typically contain at least one of anionic surfactants, amphoteric surfactants, cationic surfactants or cationic polymers and non-ionic surfactants. Such surfactants typically allow for an acceptable cleansing effect which needs to be achieved during the relatively short period of time of a bath or a shower. For typical compositions, anionic surfactants make up the bulk of the surfactant ingredients. However, anionic surfactants also seem to have the highest effect on the removal and/or damaging of skin lipids and skin proteins which are naturally present on the skin and the hair, by e.g. denaturation of the latter. Anionic surfactants are therefore considered as a main source for damaging of such components and the removal of protective skin lipids.

Residual surfactants in the hair make it look dull. It is therefore common to provide for conditioning agents in order to reduce the negative effects of the surfactant-containing cleaning compositions. Cationic surfactants and cationic polymers are typically used in conditioners. They can form thin layers on hair shafts, reducing their tendency to tangle. Furthermore, they can bind to residual anionic surfactants in order to wash them out.

It is known that the addition of a polysaccharide chosen from non-ionic or anionic fructans and starch hydrolysates has a positive effect on cosmetic properties of a shampoo. European patent application publication no. EP 1 380 284 A1 discloses that cosmetic compositions containing such a polysaccharide made for shampoos that improved hair volume and hair manageability.

It has now been found that the combination of at least one saponin and at least one saccharide polymer selected from the group consisting of starch hydrolysates, fructans, fructooligosaccharides, and mixtures thereof, in a cosmetically acceptable medium or cosmetic composition, leads to unexpected improved cosmetic properties of said composition when used in rinse-off applications (or rinse-off cosmetic products), such as shampoos, conditioners, liquid and solid soaps, shower gels and bath additives, as well as in leave-on applications (or leave-on cosmetic products) such as skin (hydration) creams, face creams, lotions, anti-ageing creams, etc.

Indeed, the inventors have surprisingly found that the compositions as described herein exhibit superior cosmetic properties, in comparison with reference compositions. For rinse-off compositions, the reference compositions are compositions that furthermore have the same ingredients in about the same concentrations, but may either substantially exclude one or both of said at least one saponin and said at least one saccharide polymer in the composition, or replace said components by an ingredient with a similar function. The comparative examples provided hereinbelow provide more details on the reference compositions that have been used in testing.

Another aspect according to the invention relates to the use of a combination of at least one saponin and at least one saccharide polymer selected from the group consisting of starch hydrolysates, fructans, fructooligosaccharides, and mixtures thereof, in a cosmetically acceptable medium, such as in cosmetic compositions. Hence, the invention relates to the use of such a composition as a cosmetic product or in the preparation of a cosmetic product.

Another aspect according to the invention relates to a method for extracting said at least one saponin from its source, in particular a plant source, by use of a mixture of water and an alcohol.

According to advantageous embodiments of the invention, the combination of said at least one saponin and said at least one saccharide polymer as described herein allows for the partial replacement of ingredients which may be of synthetic or non-natural origin or which may be perceived by the general public less favorably. More in particular, the composition allows for the partial replacement of surfactants, preferably anionic surfactants.

According to embodiments of the invention, said saccharide polymer is chosen from the group consisting of starch hydrolysates and fructans as well as mixtures thereof.

According to embodiments of the invention, the at least one saccharide polymer is present in the composition in a proportion which is at least 0.01 wt%, preferably at least 0.05 wt%, more preferably at least 0.1% by weight, even more preferably at least 0.2 wt%, more preferably at least 0.3 wt%, even more preferably at least 0.4 wt% and most preferably at least 0.5 wt%, relative to the total weight of the composition. It is further understood that the at least one polysaccharide is present in the composition in a proportion which is at most 8 wt%, preferably at most 7 wt%, more preferably at most 6 wt%, even more preferably at most 5 wt%, more preferably at most 4 wt%, even more preferably at most 3 wt%, more preferably at most 2 wt% and most preferably at most 1.5 wt%, by weight relative to the total weight of the composition.

According to an aspect of the invention, there is provided a cosmetic product comprising said cosmetic composition. Said cosmetic product may be a rinse-off cosmetic product or a leave-on cosmetic product. It will be appreciated by the skilled in the art that the concentration for said at least one polysaccharide which will be typically applied in the final product is dependent on the type of cosmetic product.

When part of a rinse-off cosmetic product, the at least one polysaccharide is present in the product in a concentration which is at least 0. 01 wt%, preferably at least 0.1 wt%, more preferably at least 0.5 wt% and most preferably at least 1 wt% with respect to the total weight of the cosmetic product. Furthermore, the at least one polysaccharide is present in the cosmetic product in a concentration which is at most 8 wt%, preferably at most 7 wt% and most preferably at most 6 wt% with respect to the total weight of the product.

When part of a leave-on cosmetic product, the at least one polysaccharide is present in the product in a concentration which is at least 0. 01 wt%, preferably at least 0.1 wt%, more preferably at least 0.5 wt% and most preferably at least 1 wt% with respect to the total weight of the cosmetic product. Furthermore, the at least one polysaccharide is present in the cosmetic product in a concentration which is at most 8 wt%, preferably at most 7 wt%, more preferably at most 6 wt%, even more preferably at most 5 wt% and most preferably at most 4 wt%, with respect to the total weight of the product.

The **starch hydrolysates** as referred hereinto are preferably characterized by a Dextrose Equivalent (DE) of 20 or less. Dextrose Equivalent (DE) refers herein to the reducing power of a starch hydrolysate expressed as a percent of the reducing power of the same weight of D-glucose. Said DE is typically measured by Lane-Eynon titration (by use of ISO 5377:1981). The DE provides a measure of the degree of hydrolysis of starch into e.g. maltodextrins (having a DE ranging from 3% to 19%) and glucose syrups (having a DE of at least 20%). The DE value of a starch hydrolysate is inversely related to the number-average degree of polymerisation (or "avDP" herein after) of the starch hydrolysate. As a rule of thumb, DE x avDP is about 120.

According to preferred embodiments of the invention, the at least one saccharide polymer is or comprises a **fructose-based polymer** (oligosaccharide or polysaccharide), i.e. a polymer of fructose molecules, which may or may not bear one terminal glucose unit. Typically, fructose-based polymers are subdivided into levan and inulin.

According to embodiments of the invention, said fructose-based polymer is of the levan type. Levan-type polymers are composed of fructooligosaccharide of polyfructose molecules wherein the fructosyl units are mostly connected to each other by β-2,6-linkages. Levan is naturally synthesized by certain plant species and by certain bacteria.

According to embodiments of the invention, said fructose-based polymer is of the inulin type. Inulin-type polymers are composed of fructooligosaccharide or polyfructose molecules of which the fructosyl units are exclusively or mainly connected to each other by β-2,1-linkages and which furthermore may or may not bear one terminal glucose unit. The molecules can be linear, namely when all the fructosyl units are exclusively connected to each other by β-2,1-linkages, but can also be branched, namely when the fructosyl units are connected to a certain extent but for less than 50 percent to each other by β-2,6-linkages. The degree of polymerization (DP) is expressed on the basis of the number of fructosyl units forming the polymer. Inulin-type polymers composed of molecules with a DP ranging from 2 to about 100,000 are commonly named "inulin". Inulin-type polymers composed of molecules with a DP ranging from 2 to 10 are commonly and interchangeably named "oligofructose", "fructo-oligosaccharide" or "inulo-oligosaccharide". The term "inulin-type polymer", as used herein, refers both to inulin and oligofructose. For the sake of completeness, it is noted that the term "inulin-type fructan" is sometimes used in literature to refer to the notion of "inulin-type polymer" as defined above.

Inulin-type polymer is naturally synthesized by many plant species but can also originate from bacterial activity. Inulin-type polymer naturally occurs as a polydisperse mixture of linear and/or branched polyfructose molecules that is characterized *inter alia* by the number-average degree of polymerization (avDP) of the polyfructose molecules. The DP and the avDP of inulin-type fructan are dependent on the origin of the fructan Typically, inulin from plant origin has a DP ranging from 2 to about 200, mostly from 2 to about 100.

Inulin is mostly obtained by isolation from roots of chicory (*Cichorium intybus*), from tubers of Jerusalem artichoke (*Helianthus tuberosus*) and Dahlia, and from Agave, preferably from the head of the Blue Agave. In the native form, namely without a treatment to increase or reduce the DP, chicory inulin has a DP ranging from 2 to about 70 and an average degree of polymerization of about 10 to about 12. Similarly, Jerusalem artichoke inulin has a DP ranging from 2 to about 40 and an average degree of polymerization of about 6; dahlia inulin an average degree of polymerization of about 15 to about 20; and agave inulin an average degree of polymerization of about 14 to about 18. Agave inulin is typically more branched than inulin isolated from chicory.

Oligofructose can be obtained at industrial scale by partial (acidic or enzymatic) hydrolysis of inulin from plant origin or from bacterial origin, or by enzymatic synthesis from saccharose according to well-known techniques (e.g., Anwar et al. Appl Environ Microbiol. 2008 Jun; 74(11): 3426-3433.). Typically, such oligofructose then has a DP ranging from 2 to about 9.

At commercial scale, inulin is mainly manufactured from chicory roots. Chicory inulin is for example available as a spray-dried powder, and can be obtained in various grades, characterized inter alia by a varying DP and average degree of polymerization.

According to preferred embodiments of the invention, the inulin-type polymer is **oligofructose.** Preferably, said oligofructose is an oligofructose with a DP which is at least 2, more preferably at least 3. It is further understood that said oligofructose has a DP which is at most 10, preferably at most 9 and more preferably at most 8.

According to alternative embodiments of the invention, the inulin-type polymer is **inulin** with a DP ranging from 11 to about 200, more preferably inulin with a DP ranging from 11 to about 100.

According to preferred embodiments of the invention, said inulin-type polymer is an inulin or oligofructose from plant origin; more preferably, the inulin-type polymer is chicory inulin. Alternatively, said inulin-type polymer is inulin from Jerusalem artichoke, inulin from Dahlia, or said inulin-type polymer is a branched inulin, for example agave inulin.

In still other alternative embodiments of the invention, the inulin-type polymer is obtained by partial (acidic or enzymatic) hydrolysis of inulin from plant origin or from bacterial origin, or by enzymatic synthesis from saccharose.

**Saponins** are a class of chemical compounds which can be structurally defined in that they consist of an aglycone unit (or "sapogenin unit"), derived from a lipophilic triterpene or sterol unit, linked to one or more hydrophilic glycoside moieties. The sapogenin unit's carbon skeleton may be saturated or unsaturated and/or comprise a heteroatom such as nitrogen. The glycoside moiety contains sugars such as galactose, glucose, glucuronic acid, methyl pentose, rhamnose, and xylose. Due to the hydrophilic nature of the glycoside moieties, as well as the lipophilic nature of the triterpene or sterol unit, saponins possess surface-active properties. Saponins are further known to have to a greater or lesser extent an antimicrobial, antiherbivore and/or cytotoxic activity. Their role in nature is likely to be in the defense against pathogens, pests and predators.

Saponins may be obtained from natural sources. For the purpose of the invention, the term "natural" as in "natural component" refers to the situation wherein such a component contained in the composition is obtained from a natural source (plant source or non-plant source, e.g. animal source or marine organisms) in a substantially unmodified form, namely in the form which exists in the material of natural origin. Referring specifically to the category of marine organisms, it is known that saponins may be obtained *inter alia* from sea cucumbers and starfish.

Saponins are considered as non-ionic surfactants although they can have a negative charge due to the presence of one or more carboxylic groups.

An aspect of the invention relates to a process for preparing a cosmetic composition as described above, the process comprising: extracting at least one saponin from its source using a mixture of water and a water-soluble alcohol; and combining said at least one saponin with at least one saccharide polymer selected from the group consisting of fructooligosaccharides, fructans, galactooligosaccharides, galactans, glucooligosaccharides, glucans, pectin, xylooligosaccharides, xylans, and mixtures thereof.

According to preferred embodiments of the invention, the saponins used herein are obtained from a plant source or a mixture of different sources, such as multiple plant sources.

Said plant source may be tea (including *Camellia sinensis* and *Camelia oleifeira*), species from the *Sapindus* Genus (including *Sapindus delavayi, Sapindus oahuensis, Sapindus mukorossi, Sapindus marginatus, Sapindus saponaria, Sapindus trifoliatus, Saponaria officinalis*), *Quillaja saponaria,* species of the Genus *Yucca* (including *Yucca Schidigera*), species of the Genus *Gynostemma,* shikakai, soybeans, beans, mung beans, peas (*Pisum sativum*), alfalfa, spinach, sugar beet, quinoa, liquorice, sunflower, horse chestnut, species from the Genus *Panax* (including *Panax quinquefolius, Panax japonicus*), oats, capsicum peppers, eggplant (aubergine), tomato seed, species of the *Allium* Genus, asparagus, yam, fenugreek, *Bupleurum falcatum, Desmodium adscendens, Gypsophila,* and *Styrax japonica.*

For the purpose of extracting the saponin material, any part of the plant may be used, including leaves, stems, roots, bulbs, blossom and fruit (including the skin, flesh and seed of the fruit).

According to preferred embodiments, said plant source is tea (preferably *Camelia sinensis* or *Camellia oleifera*), *Quillaja Saponaria* or a species of the *Sapindus* Genus, and said at least one saponin comprises a tea saponin or saponin from *Quillaja Saponaria* or at least one species of the *Sapindus* Genus, or mixtures thereof.

According to embodiments, said at least one saponin is a tea saponin or a mixture of saponins from *Camelia sinensis* or *Camellia oleifera.*

According to embodiments, said at least one saponin is a saponin from at least one species of the *Sapindus* Genus, or mixtures thereof.

According to embodiments, said at least one saponin is a saponin from *Quillaja Saponaria.*

According to embodiments of the invention, the at least one saponin is present in the composition in a proportion which is at least 0.001 wt%, preferably at least 0.005 wt%, more preferably at least 0.01% by weight, even more preferably at least 0.05 wt%, more preferably at least 0.1 wt%, even more preferably at least 0.2 wt% and most preferably at least 0.3 wt%, relative to the total weight of the composition. It is further understood that the at least one saponin is present in the composition in a proportion which is at most 15 wt%, preferably at most 12 wt%, more preferably at most 9 wt%, even more preferably at most 8 wt%, more preferably at most 7 wt%, even more preferably at most 6wt%, and most preferably at most 5 wt%, by weight relative to the total weight of the composition.

As mentioned here above, said cosmetic composition may be part of a cosmetic product. Said cosmetic product may be a rinse-off cosmetic product or a leave-on cosmetic product. It will be appreciated by the skilled in the art that the concentration for said at least one saponin which is typically applied in the final product will depend on the type of cosmetic product.

When part of a rinse-off cosmetic product, the at least one saponin is present in the product in a concentration which is at least 0. 01 wt%, preferably at least 0.1 wt%, more preferably at least 0.5 wt%, even more preferably at least 1 wt%, more preferably at least 2 wt%, even more preferably at least 3 wt%, more preferably at least 4wt%, and most preferably at least 5 wt% with respect to the total weight of the cosmetic product. Furthermore, the at least one saponin is present in the cosmetic product in a concentration which is at most 15 wt%, preferably at most 14 wt%, more preferably at most 13 wt%, even more preferably at most 12 wt%, more preferably at most 11 wt%, even more preferably at most 10 wt% and most preferably at most 9 wt% with respect to the total weight of the product.

When part of a leave-on cosmetic product, the at least one saponin is present in the product in a concentration which is at least 0. 001 wt%, preferably at least 0.005 wt%, more preferably at least 0.01 wt%, even more preferably at least 0.05wt%, more preferably at least 0.1 wt% and most preferably at least 0.2 wt% with respect to the total weight of the cosmetic product. Furthermore, the at least one saponin is preferably present in the cosmetic product in a concentration which is at most 5 wt%, more preferably at most 4.5 wt %, even more preferably at most 4 wt%, more preferably at most 3.5 wt%, even more preferably at most 3 wt%, more preferably at most 2.5 wt%, even more preferably at most 2 wt%, and most preferably at most 1.5 wt% with respect to the total weight of the product.

As rinse-off compositions typically have a surfactant concentration which may exceed 15 wt% with respect to the total weight of the product, it will be appreciated that the concentration of the at least one saponin will be relatively high when present in rinse-off compositions or products in comparison to leave-on products or compositions, where the at least one saponin will be used in more limited amounts in its capacity as an emulsifier. Moreover, in embodiments of the invention, it becomes possible to use the at least one saponin at lower concentrations than for a typical emulsifier while still resulting in a stable emulsion.

In a product having the form of an emulsion the hydrophobic phase typically comprises at least one oil and/or at least one other component. Said at least one other component can be selected from fatty substances, hydrophobic particles and/or waxes. For the purpose of the present invention, the term "oil" refers to a substance which is liquid at ambient temperature, and which is hydrophobic. Said oils can be mineral oils, vegetable oils or silicone oils.

In a preferred embodiment, the hydrophobic phase is an oily phase, comprising an oil, by preference selected from the group consisting of jojoba oil, isoamyl laurate, iso-hexadecane cyclopentasiloxane and mixtures thereof. Hence, according to embodiments of the cosmetic composition of the invention, said cosmetic composition forms an emulsion further comprising at least one of jojoba oil, isoamyl laurate and cyclopentasiloxane.

In the case an oily phase is dispersed in an aqueous phase, such emulsion is called an "oil-in-water emulsion" or "o/w emulsion". When the aqueous phase is dispersed in the oily phase, such emulsion is called a "water-in-oil emulsion" or "w/o emulsion".

According to embodiments of the invention, said cosmetic composition forms an o/w emulsion.

Multiple emulsions are a specific type of emulsions whereby two types can be considered, oil in water in oil (o/w/o) and water in oil in water (w/o/w).

In the case of an o/w/o emulsion, firstly an oil-in-water emulsion is prepared by using an appropriate emulsifier, comprising a saponin or an emulsifier with a high hydrophilic lipophilic balance (HLB) number. This o/w emulsion will then be emulsified in an oil phase with an appropriate w/o emulsifier with a low HLB number.

In case of a w/o/w emulsion, firstly a water-in-oil emulsion is prepared with w/o emulsifier having a low HLB number. This w/o emulsion will then be emulsified in water, together with an appropriate o/w emulsifier, comprising a saponin or an emulsifier with a high HLB number.

According to alternative embodiments of the invention, said cosmetic composition forms the o/w emulsion of an o/w/o emulsion or the o/w emulsion of an w/o/w emulsion.

According to preferred embodiments of the invention, the cosmetic composition forms a stable emulsion, referring to the ability of an emulsion to resist change in its properties over time. There are three types of instability in emulsions: irreversible flocculation, coalescence, and Ostwald ripening. Ostwald ripening is generally found in water-in-oil emulsions, while flocculation is found in oil-in-water emulsions.

Flocculation occurs when there is an attractive force between the droplets of the dispersed phase, said droplets forming flocs as a consequence, like bunches of grapes.

Coalescence occurs when droplets bump into each other and combine to form a larger droplet, so the average droplet size increases over time. In the case of solid phase emulsions, the term irreversible flocculation is used. This is applicable for example for emulsion polymers and pigments.

Ostwald ripening is the process of disappearance of small droplets by dissolution and deposition on the larger particles or droplets, as caused by the solubility differential between smaller and larger droplets.

Emulsions can also undergo creaming, whereby, in the particular case of o/w emulsions, the droplets of the dispersed phase rise to the top of the emulsion under the influence of buoyancy, or under the influence of the centripetal force induced when a centrifuge is used. It should be noted that creaming does not implicate coalescence and vice versa. Creaming, being a phase separation caused by oil droplets moving towards the top of the system, and/or sedimentation is not considered herein as an instability. Since the speed of creaming is influenced by the viscosity of the continuous phase and the droplet diameter, following the law of Stokes, large oil droplets in a macroemulsion will move faster to the top than the smaller oil droplets. It also means that if the viscosity of the continuous phase is high enough, creaming will not occur, even when the droplets are big. Increasing the viscosity of an emulsion can easily be obtained by the addition of thickening agents. In the particular case of a nanoemulsion, with oil droplets smaller than 500 nm, the phenomenon of creaming may disappear since the Brownian movement of the droplets become more important than the gravity forces.

On the other hand, nanoemulsions are more sensitive for Ostwald ripening than macroemulsions, since the speed of Ostwald ripening is in function of the droplet diameter, which is in the case of a nanoemulsion several times smaller than in the case of a macroemulsion.

An appropriate "surface active agent" (or "surfactant" or "tensioactive" or "emulsifier") can increase the kinetic stability of an emulsion so that the size of the droplets does not change significantly with time. It is then said to be stable. This means that an emulsion can be called stable when neither coalescence nor Ostwald ripening occurs in a certain period of time and at a given temperature.

For the purpose of the remainder of this description, an operational definition of emulsion stability is implied, whereby an emulsion is considered stable if it does not give rise to any of the above mentioned three types of instability after uninterrupted storage in an oven for at least 4 weeks at 50 °C.

Some compounds/molecules are known to be difficult to incorporate in emulsions since they cause coalescence. This coalescence can occur immediately after the emulsion has been prepared or in a period shorter than the desired stability requirements or at a temperature lower than the desired stability requirement. Such molecules/compounds will further be called "emulsion breakers". As examples can be given; electrolytes, cationic molecules including cationic surfactants, medium-chain length linear vicinal diols, reducing agents like potassium thioglycolate, oxidizing agents like hydrogen peroxide, sunless tanning agents like dihydroxy acetone (DHA).

The present invention does not exclude the presence of additional components in the cosmetic composition, other than the at least one saponin and the at least one saccharide polymer. According to embodiments of the cosmetic composition according to the present invention, additional components comprise one or more compounds from the following classes: detergents and anti-bacterial agents; surfactants, more in particular anionic, non-ionic, amphoteric and/or cationic surfactants; cleansing agents, preservatives and pH-stabilizing agents, lather-forming agents, conditioning agents, thickening agents, coloring agents, perfuming agents, antioxidants, plant extracts, and combinations thereof. Such compounds are well known in the state of the art.

For the preparation of an emulsion with a certain stability, there is a need for the presence of a surface-active molecule that can lower the interfacial tension of the two non-miscible liquids together with the input of mixing energy that can be provided through shaking, stirring and/or homogenizing.

According to embodiments of the invention, said cosmetic composition further comprises at least one surfactant, other than saponin.

Surfactants are compounds that lower the surface tension (or interfacial tension) between two liquids or between a liquid and a solid. They are usually organic compounds that are amphiphilic, meaning they contain both hydrophobic groups (their tails) and hydrophilic groups (their heads). Therefore, a surfactant contains both a water-insoluble (or oil-soluble) component and a water-soluble component. Surfactants will diffuse in water and adsorb at interfaces between air and water or at the interface between oil and water, in the case where water is mixed with oil. The water-insoluble hydrophobic group may extend out of the bulk water phase, into the air or into the oil phase, while the water-soluble head group remains in the water phase. Commonly encountered surfactants typically belong to one of the following classes: anionic, cationic, amphoteric (zwitterionic) and non-ionic.

According to embodiments of the invention, said cosmetic composition further comprises at least one of a non-ionic surfactant other than saponin, an anionic surfactant, a cationic surfactant and an amphoteric surfactant.

According to embodiments of the invention, the cosmetic composition further comprises at least one non-ionic surfactant, which is selected from the following:

- fatty alcohols, such as cetyl alcohol, stearyl alcohol, and cetostearyl alcohol (consisting predominantly of cetyl and stearyl alcohols), and oleyl alcohol;
- non-ionic surfactants whereby the alcohol function has been substituted by a variety of different polar groups, such as polyethylene glycol alkyl ethers like octaethylene glycol monododecyl ether and pentaethylene glycol monododecyl ether, polypropylene glycol alkyl ethers, glucoside alkyl ethers like decyl glucoside, lauryl glucoside and octyl glucoside, sucrose alkyl esters, carbohydrate alkyl carbamates like inulin lauryl carbamate, glycerol alkyl esters like glyceryl laurate, glyceryl mono-stearate, polyoxyethylene glycol sorbitan alkyl esters like polysorbate, sorbitan alkyl esters like Spans, cocamide MEA, cocamide DEA, dodecyldimethylamine oxide, block copolymers of polyethylene glycol and polypropylene glycol like poloxamers and polyethoxylated tallow amine (POEA);
- ethoxylated fatty acids.

According to embodiments of the invention, the cosmetic composition further comprises at least one anionic surfactant, which is selected from the following:

- anionic surfactants containing anionic functional groups at their head, such as sulfate, sulfonate, sulfosuccinates, sarcosinates, glycine, glutamate phosphate, and carboxylates;
- alkyl sulfates including ammonium lauryl sulfate, sodium lauryl sulfate (sodium dodecyl sulfate, SLS, or SDS), and their related alkyl-ether sulfates, such as sodium laureth sulfate (sodium lauryl ether sulfate or SLES).

According to embodiments of the invention, the cosmetic composition further comprises at least one cationic surfactant, which is selected from the following:

- cationic surfactants with permanently charged quaternary ammonium salts including cetrimonium bromide (CTAB), cetrimonium chloride, cetylpyridinium chloride (CPC), behentrimonium chloride, behentrimonium methasulphate, stearyl dimonium hydroxypropyl laurylglucosides chloride

However, mostly these cationic surfactants are difficult to incorporate in emulsions and can be considered as emulsion breakers.

Zwitterionic (amphoteric) surfactants have both cationic and anionic centers attached to the same molecule. The cationic part is based on primary, secondary, or tertiary amines or quaternary ammonium cations. The anionic part can be more variable.

According to embodiments of the invention, the cosmetic composition further comprises at least one amphoteric surfactant, which is selected from the following:

- certain sulfonates, including the sultaines CHAPS (3-[(3-Cholamidopropyl)-dimethylammonio]-1-propanesulfonate) and cocamidopropyl hydroxysultaine;
- betaines such as cocamidopropyl betaine having a carboxylate with the ammonium;
- amphoteric surfactants having a phosphate anion with an amine or ammonium, such as the phospholipids phosphatidylserine, phosphatidylethanolamine, phosphatidylcholine, and sphingomyelins.

According to embodiments of the invention, saponins may be used as a surface-active agent only, as an emulsifier only, or as both; saponins may be used alone or in combination with other surfactants/emulsifiers/tensio-actives.

Preferably, the weight ratio of said at least one saponin and the total of all surfactants in the composition, excluding said at least one saponin, is at least 1:100, preferably at least 1:90, more preferably at least 1:80, even more preferably at least 1:70, more preferably at least 1:60, even more preferably at least 1:50, more preferably at least 1:40 and most preferably at least 1:30. It is further understood that said weight ratio is at most 100:1, preferably at most 70:1, more preferably at most 30:1, even more preferably at most 10:1, more preferably at most 1:1, even more preferably at most 1:2, more preferably at most 1:5 and most preferably at most 1:7.

According to embodiments of the invention, said cosmetic composition further comprises one or more emulsion breaking agents. Preferably, said one or more emulsion breaking agents are selected from:

- an antimicrobial agent such as a medium-chain length linear vicinal diols, glyceryl caprylate, pentylene glycol, or caprylyl glycol;
- an electrolyte such as $MgSO_4$, or NaCl;
- a cationic molecule such as cationic surfactants such as cetrimonium chloride;
- an oxidising agent such as hydrogen peroxide up to concentration between 10 and 30%;
- a sunless tanning agent such as dihydroxy acetone (DHA);
- reducing agents such as potassium thioglycolate;
- alfa hydroxy acids such as glycolic acid;
- alpha and beta hydroxy acids, such as salicylic acid;
- surfactants, such as cocamidopropyl betaine, sodium cocoyl glutamate, or sodium lauroyl glutamate;
- $C_1$-$C_7$ alcohols, such as ethanol; and
- perfume compounds.

It was found that emulsions according to the invention remained stable even after addition of specific amounts of one of said emulsion breaking agents. This unexpected effect is further illustrated in Experiment 14, described below, for typical concentrations of emulsion breaking agents.

According to embodiments of the invention, the cosmetic composition forms an emulsion wherein the hydrophilic phase forms an aqueous phase, comprising water.

Preferably, the hydrophilic phase contains water and hydrophilic adjuvants, including monoalcohols containing 2 to 8 carbon atoms, for instance ethanol and isopropanol; and polyols, for instance glycerol; glycols, for instance pentylene glycol, propylene glycol, butylene glycol, isoprene glycol and polyethylene glycols such as PEG-8; sorbitol; sugars such as glucose, fructose, maltose, lactose or sucrose, and mixtures thereof.

However, lower alcohols, such as ethanol are also known and used as emulsion breaking agent.

The polyol that is miscible with water at room temperature (25 °C) may be chosen from polyols containing 2 to 20 carbon atoms, preferably containing 2 to 10 carbon atoms and more preferably containing 2 to 6 carbon atoms, such as glycerol, propylene glycol, butylene glycol, pentylene glycol, hexylene glycol, dipropylene glycol or diethylene glycol; glycol ethers (especially containing 3 to 16 carbon atoms), such as mono-, di- or tripropylene glycol (C1-C4) alkyl ethers or mono-, di- or triethylene glycol (C1-C4) alkyl ethers, and mixtures thereof.

According to embodiments of the invention, the cosmetic composition forms an emulsion wherein the hydrophobic phase comprises at least one oil and/or at least one other component.

Said at least one other component can be selected from fatty substances, hydrophobic particles and/or waxes.

Mention may be made especially of oils, fatty esters, waxes and butters, which may be, respectively, of natural (animal or plant) origin or synthetic origin.

Fatty substances of natural origin will preferentially be used, such as plant oils, fatty esters of plant origin and waxes or butters of plant origin.

Fatty substances can also be gums and pasty fatty substances, of animal, plant, mineral or synthetic origin, and mixtures thereof.

The oily phase may also comprise any common liposoluble or lipodispersible additive.

Preferably, the hydrophobic phase contains at least one cosmetic oil.

According to embodiments of the invention, said oil is a volatile oil.

The term "volatile oil" means any non-aqueous medium that is capable of evaporating from the skin or the lips in less than one hour, especially having a vapor pressure, at room temperature and atmospheric pressure, ranging from $10^{-3}$ to 300 mmHg (0.13 Pa to 40 000 Pa). As volatile oils that may be used in the invention, it is possible to use non-silicone volatile oils, especially $C_8$-$C_{16}$ isoparaffins, for instance isododecane, or isodecane.

According to embodiments of the invention, said oil is a non-volatile oil.

The term "non-volatile oil" means an oil that is capable of remaining on the skin at room temperature (25 °C) and atmospheric pressure for at least one hour and that especially has a non-zero vapor pressure at room temperature (25 °C) and atmospheric pressure, of less than 0.01 mmHg (1.33 Pa).

As non-volatile oils that may be used in the invention, mention may be made of non-silicone and especially hydrocarbon-based non-volatile oils, such as liquid paraffin (or petroleum jelly), squalane, hydrogenated polyisobutylene (parleam oil), perhydrosqualene, mink oil, soybean oil, sweet almond oil, beauty- leaf oil, palm oil, grapeseed oil, sesame seed oil, corn oil, arara oil, rapeseed oil, sunflower oil, cottonseed oil, apricot oil, castor oil, avocado oil, jojoba oil, olive oil, argan oil, virgin sweet almond oil, apricot kernel oil, rice bran oil, camellia oil or cereal germ oil; as preferred oils, jojoba oil or apricot kernel oil or any other triglyceride, and mixtures thereof, will be used; lanolic acid, oleic acid, lauric acid or stearic acid esters; esters derived from long-chain acids or alcohols (i.e. chains containing from 6 to 20 carbon atoms), such as isoamyl laurate, especially the esters of formula RCOOR' in which R represents a higher fatty acid residue containing from 7 to 19 carbon atoms and R' represents a hydrocarbon-based chain containing from 3 to 20 carbon atoms, in particular C12-C36 esters, such as isopropyl myristate, isopropyl palmitate, butyl stearate, hexyl laurate, diisopropyl adipate, isononyl isononanoate, 2-ethylhexyl palmitate, 2-hexyldecyl laurate, 2-octyldecyl palmitate, 2-octyldodecyl myristate or lactate, bis (2-ethylhexyl) succinate, diisostearyl malate, or glyceryl or diglyceryl triisostearate,- higher fatty acids, especially of C14- C22 such as myristic acid, palmitic acid, stearic acid, behenic acid, oleic acid, linoleic acid, linolenic acid or isostearic acid; higher fatty alcohols, especially of C16-C22 such as cetanol, oleyl alcohol, linoleyl alcohol, linolenyl alcohol, isostearyl alcohol or octyldodecanol; and mixtures thereof.

According to embodiments of the invention, said oil is a silicone oil.

Silicone oils, for instance volatile or non-volatile polymethylsiloxanes (PDMSs) containing a linear or cyclic silicone chain, which are liquid or pasty at room temperature, especially volatile silicone oils, in particular, cyclopentasiloxane, cyclopolydimethylsiloxanes (cyclomethicones) such as cyclohexadimethylsiloxane and cyclopentadimethylsiloxane; polydimethylsiloxanes comprising alkyl, alkoxy or phenyl groups, which are pendent or at the end of a silicone chain, these groups containing from 2 to 24 carbon atoms; phenyl silicones, for instance phenyl trimethicones, phenyl dimethicones, phenyltrimethylsiloxydiphenylsiloxanes, diphenyl dimethicones, diphenylmethyldiphenyltrisiloxanes, 2-phenylethyltrimethylsiloxysilicates and polymethylphenylsiloxanes cross-polymers with vinyl groups and alkyl groups, such as vinyl dimethicone crosspolymer, $C_{30}$-$C_{45}$ alkyl cetearyl dimethicone crosspolymer or polymers such as polymethylsilsesquioxane, and mixtures thereof.

The choice of the oils is especially made as a function of the desired aim. Thus, the triglycerides and the plant oils such as apricot oil or olive oil are preferred for compositions intended for cosmetic application on dry skin, whereas fatty acid esters, which are lighter, are preferred for compositions intended for normal or combination skin.

Other fatty substances that may be present in the oily phase are, for example, fatty acids containing from 8 to 30 carbon atoms, for instance stearic acid, lauric acid or palmitic acid; pasty fatty substances, for instance petroleum jelly or lanolin.

Similarly, the oily phase may comprise a fat which is a derivative of a fatty acids such as described above and glycerol. Examples fats used in cosmetics comprise butters such as Sheabutter, Cupuacu butter and Mango butter.

Waxes are organic compounds that characteristically consist of long alkyl chains. They may also include various functional groups such as fatty acids, primary and secondary long chain alcohols, unsaturated bonds, aromatics, amides, ketones, and aldehydes. They frequently contain fatty acid esters as well. Synthetic waxes are often long-chain hydrocarbons (alkanes or paraffins) that lack functional groups.

Examples thereof are waxes, for instance beeswax, carnauba wax, candelilla wax, paraffin wax, lignite wax, microcrystalline waxes, ceresin, ozokerite, synthetic waxes such as polyethylene waxes, polymethylene waxes and Fischer-Tropsch waxes.

Said substances may be chosen in a varied manner by a person skilled in the art in order to prepare a composition having the desired properties, for example in terms of consistency or texture.

According to embodiments of the invention, the cosmetic composition comprises at least one cosmetic adjuvant chosen from cosmetically active agents, preserving agents, antioxidants, fragrances, fillers, UV-screening agents, pigments, odor absorbers and dyestuffs.

Typical active agents are moisturizers; free-radical scavengers; keratolytic and desquamating agents; vitamins; anti-elastase and anti-collagenase agents; trace elements; algal or plankton extracts; enzymes and coenzymes; flavonoids and isoflavonoids; ceramides; anti-glycation agents; NO- synthase inhibitors; agents for stimulating the synthesis of dermal or epidermal macromolecules and/or for preventing their degradation; agents for stimulating fibroblast or keratinocyte proliferation and/or keratinocyte differentiation; tensioning agents; anti-pollution agents and/or free-radical scavengers; muscle relaxants or dermo-decontracting agents, and mixtures thereof.

More in particular, examples of active agents that may be mentioned include (N-2-hydroxyethylpiperazine-N-2-ethane) sulfonic acid (HEPES); hyaluronic acid; lanolin; urea which is a typical emulsion breaker, and mixtures containing urea such as NMF (Natural Moisturizing Factor), and urea derivatives such as N-(2-hydroxy- ethyl)urea (Hydrovance from the company National Starch); 2-oxothiazolidine-4-carboxylic acid (procysteine); $\alpha$-hydroxy acids, especially fruit-based acids, for instance glycolic acid, which is a typical emulsion breaker, lactic acid, malic acid, citric acid, tartaric acid or mandelic acid, and derivatives and mixtures thereof; $\beta$-hydroxy acids, for instance salicylic acid and its derivatives such as 5-n-octanoylsalicylic acid or 5-n-dodecanoylsalicylic acid; $\alpha$-keto acids, for instance ascorbic acid or vitamin C and its derivatives such as its salts, for instance sodium ascorbate and magnesium or sodium ascorbyl phosphate; its esters, for instance ascorbyl acetate, ascorbyl palmitate and ascorbyl propionate, its ethers, for instance ethyl ascorbic acid, or its sugars, for instance glycosyl ascorbic acid, and mixtures thereof; $\beta$-keto acids,- retinoids, for instance retinol (vitamin A) and its esters, retinal, retinoic acid and its derivatives, and also the retinoids, carotenoids such as lycopene,- ceramides; resveratrol; pseudodipeptides such as {2-[acetyl (3-trifluoromethylphenyl) amino] -3-methylbutyryl- amino} acetic acid; vitamins, for instance, besides vitamins A and C indicated above, vitamin E (tocopherol), vitamin B3 (or vitamin PP or niacinamide), vitamin B5 (panthenol in its various forms: D-panthenol, DL-panthenol), vitamin D and vitamin F (mixture of essential fatty acids), and derivatives, precursors and analogues of these vitamins; soybean extracts, in particular soybean protein hydrolysates or isoflavone-rich soybean extracts; trace elements, for instance copper, zinc, selenium, iron, magnesium or manganese; algal extracts, plankton extracts such as the plankton in aqueous dispersion (CTFA name: Vitreoscilla ferment), enzymes, coenzymes such as ubiquinone or coenzyme QIO which belongs to the family of benzoquinones containing an alkylene chain, coenzyme R, which is biotin (or vitamin H); yeast extracts, for instance the extract of S. cerevisiae, adenosine; plant extracts, for instance extracts of liquorice; calmatives, for instance bisabolol and calmative plant extracts, for instance extracts of rose (Rosa gallica) and extracts of mint (Mentha piperita); and any active agent that is suitable for the final aim of the composition, and mixtures thereof.

The ingredients and/or active agents may range from 0.01% to 20% by weight, 0.05% to 10% or from 0.1% to 1% by weight relative to the total weight of the composition.

According to embodiments of the invention, the cosmetic composition comprises at least one UV-screening agent, preferably at least one lipophilic or liposoluble UV-screening agent. Examples of lipophilic UV-screening agents include anthranilates; cinnamic derivatives; dibenzoylmethane derivatives; salicylic derivatives; camphor derivatives; triazine derivatives; benzophenone derivatives; $\beta,\beta$-diphenyl-acrylate derivatives; benzotriazole derivatives; benzalmalonate derivatives, benzimidazole derivatives; imidazolines; p-aminobenzoic acid (PABA) derivatives; benzoxazole derivatives; screening polymers; $\alpha$-alkylstyrene-based dimers; 4,4-diarylbutadienes and mixtures thereof. Lipophilic UV-screening agents are typically present in concentrations ranging from 0.1% to 30% by weight or 0.5% to 15% by weight relative to the total weight of the composition.

According to embodiments of the invention, the cosmetic composition comprises at least one filler. Examples of fillers include powders of natural organic materials such as corn, wheat or rice starch; or alternatively materials of natural mineral origin, for instance silica, talc, clays, for instance kaolin, montmorillonite, saponites, laponites and illites. Fillers may be present at less than or equal to 20% of the total weight of the composition, less than or equal to 10% of the total weight of the composition, less than or equal to 8%, or less than or equal to 5% of the total weight of the composition. When they are present, these fillers may be present in amounts ranging, for example, from 0.05% to 8% by weight or from 0.1% to 5% by weight relative to the total weight of the composition.

According to embodiments of the invention, the cosmetic composition further comprises hydrophobic particles such as pigments that can be coated with silicones such as triethoxycaprylylsilane, dimethicone, dimethicone copolyol, perfluorooctyl triethoxysilane, trimethylsiloxysilicate, esters such as isopropyl titanium triisostearate, sodium glycerophosphate, magnesium myristate, polyperfluoromethylisopropyl ether, hydrogenated lecithin, lauroyl lysine, blends based on natural waxes, such as carnauba wax and polyethylene wax, and or vegetable oils and esters such as jojoba ester, and mixtures thereof.

Said hydrophobic particles may serve as cosmetic preservation products.

In recent years cosmetic, toiletry and pharmaceutical manufacturers have been severely limited in their choice of preservative agents. One class of biocides that has been highly effective in cosmetic, toiletry and pharmaceutical products includes formaldehyde donors, such as imidazolidinyl urea, diazolidinyl urea, and DMDM hydantoin. However, many such compounds are considered to be skin irritants and the use of formaldehyde donors is severely restricted by regulations in the EU and Japan.

Another class of preservatives includes the isothiazolinones. Methylchloroisothiazolinone is a chloro-substituted isothiazolinone that has demonstrated irritation potential and it is prohibited from use in leave-on products in some countries.

Another class of preservatives is chlorinated aromatic compounds, such as chlorphenesin. They are not broadly used in cosmetic, toiletries or pharmaceuticals because they exhibit a very strong and unpleasant odor. Also chlorinated compounds in general are used in herbicides and pesticides, and many are known human toxins, and thus chlorinated compounds may have a negative consumer perception.

Yet another class of preservatives is para-hydroxybenzoic acids, known as parabens. Preservative blends containing parabens, are the most widely used preservative systems and have been used safely and effectively for over 20 years. However, some research has suggested that parabens are possible human carcinogens. The media has suggested that products containing parabens are dangerous. Consumer groups, such as Breast Cancer Action, have lobbied cosmetic and toiletry companies to remove parabens from their products. As a result, parabens are now *de facto* banned from many segments of the cosmetic and toiletry industry.

Alcohols, and in particular diols, more specifically vicinal diols are attractive alternatives for the above preservatives. "Vicinal diols", as used herein, are materials that have hydroxyl groups which are bonded to atoms in the molecule which are next to each other, i.e., wherein two atoms each bearing a hydroxyl group are bonded to each other. Examples of vicinal diol compounds include, but are not limited to, ethylene glycol and propylene glycol. Such materials are known for use as humectants and solvents in cosmetic, toiletry and pharmaceutical products. They are also known to have some modest antimicrobial activity as described in U.S. patent application publication no. US 2007/0207105 A1, the disclosure of which is incorporated herein by reference to the extent that pertains to vicinal diols and compositions incorporating these compounds.

The most preferred vicinal diols for use in the compositions described herein when used in cosmetic, toiletry and pharmaceutical applications are medium-chain length, linear vicinal diols that demonstrate antimicrobial activity at relatively low use-levels. Such diols include 1,2-pentanediol, 1,2-hexanediol, caprylyl glycol, and 1,2-decanediol. Other vicinal diols useful in the compositions described herein include molecules derived from glycerin. Glycerin can be reacted with other molecules at its 1- or 3-position, leaving two vicinal hydroxyl groups. For example, glyceryl monoethers, such as ethylhexylglycerin [3-(2-ethylhexyloxy)propane-1,2-diol], are useful liquid vicinal diols having antimicrobial properties. Glyceryl monoesters such as glyceryl monolaurate, glyceryl mono caproate, or glyceryl monocaprylate, are also useful antimicrobial vicinal diols. For the preservation of cosmetics, toiletries and pharmaceuticals, vicinal diols are known to be effective against bacteria and yeast but weak against fungi. However, above vicinal diols, such as caprylyl glycol and glyceryl monocaprylate are typical emulsion breakers.

In general, preservatives that can be present in cosmetic formulations are listed in EC1223/2009 Annex V.

According to embodiments of the present invention, the cosmetic composition further comprises hydrophobic particles and/or alcohols such as vicinal diols and are also substantially free of parabens.

Other organic acids are anisic acid and its salts, levulenic acid and its salts, dehydroacetic acid and its salts.

It is known that the presence of molecules with electrolyte properties, e.g. salts or other molecules which have a positive or negative charge, dependent on the solubility and the pH, may induce coalescence of an emulsion, because of the dehydrating effect of the surface-active agents.

Electrolytes are a thus considered to be emulsion breakers. On the other hand, the presence of charged molecules can be desired because of its advantages for the final formulation.

According to embodiments of the present invention, the cosmetic composition further comprises at least one electrolyte.

One can use a mixture of salts, including natural mixtures or mixtures whose composition is close to that of a natural mixture, especially an aqueous mixture comprising 3 to 30 wt% magnesium chloride, 0.2-10 wt% magnesium sulfate, from 2 to 28 wt% of potassium chloride, 3 to 30 wt% sodium chloride, 0.2 to 10 wt% of calcium chloride, 0.1 to 6 wt% magnesium bromide and 0.1 to 0.5 wt% insolubles, relative to the total weight of all components, said mixture being referred to herein as "the mixture of Dead Sea salts" (Dead Sea bath salts) as it corresponds to the main salts contained in the Dead Sea.

The salts may also be in the form of a solution, in particular as a thermal or mineral water. In general, a mineral water is suitable for consumption, which is not always the case with a thermal water. Each of these waters contains, inter alia, dissolved minerals and trace elements.

Electrolytes may also be chosen from the active salts, for example salts derived from vitamins such as vitamin C (ascorbic acid) or acid active salts as acid salts filters. Salts derived from vitamins include e.g. ascorbyl phosphate of an alkali metal, alkaline earth or transition as magnesium, sodium, potassium, calcium, zinc; retinyl phosphate of an alkali or alkaline earth metal such as magnesium, potassium.

Salts derived filters, comprise benzenesulfonic acid salts of 1, 4-[di (3-methylidene-10-sulphonic acid)], the sodium sulphonate of 2-hydroxy-4-methoxy benzophenone-5 (or benzophenone-5), the disodium salt of 2,2'-dihydroxy-4,4'-dimethoxy-5,5'-disulfo-benzophenone (benzophenone or 9).

To balance skin feel and functionality, many formulators use PBSA, 2-phenylbenzimidazole-5-sulfonic acid, a water-soluble sunscreen with excellent absorption in the UV-B wavelength region, in combination with oil-soluble UV filters. However, PBSA must be neutralized to pH 7-8 prior to use or it may revert to its acid form and crystallize, rendering it less effective as a sunscreen active. At pH 7-8, it is available in its salt form, which will negatively interact with ionic polymers, resulting in low viscosity.

In certain embodiments of the present invention, the compositions may comprise a thickener, for instance to reduce or prevent creaming. Creaming refers herein to a phase separation, typically caused by oil droplets moving towards the top of the system, but which does not implicate coalescence.

Many thickeners are polysaccharides of natural origin or plant origin. "Polysaccharides of plant origin" especially refers to polysaccharides obtained from the plant kingdom (plants or algae), as opposed to polysaccharides obtained via biotechnology, as is the case, for example, for xanthan gum, which is produced especially by fermentation of a bacterium, *Xanthomonas campestris.*

A plant-derived polysaccharide may, where appropriate, be chemically modified to promote its hydrophilic valency, as is the case for cellulose derivatives, in particular hydroxyalkyl celluloses (e.g.: hydroxyethylcellulose).

Suitable examples of polysaccharides of plant origin include algal extracts, such as alginates, carrageenans and agars, and mixtures thereof, gums, such as guar gum and nonionic derivatives thereof (hydroxypropyl guar), gum arabic, konjac gum or mannan gum, gum tragacanth, ghatti gum, karaya gum or locust bean gum, modified or unmodified starches, such as those obtained, for example, from cereals, for instance wheat, corn or rice, from legumes, for instance blonde pea, from tubers, for instance potato or cassava, and tapioca starches; dextrins, such as corn dextrins, celluloses and derivatives thereof, in particular alkyl celluloses, hydroxyalkyl celluloses; and alkyl hydroxyalkyl celluloses; mention may be made especially of methyl-celluloses, hydroxyethylcelluloses, ethyl-hydroxyethylcelluloses and carboxymethyl-celluloses. Examples that may be mentioned include stearyl and cetyl hydroxyethylcellulose.

Other suitable examples of polysaccharides are linear polyglucoses obtained by fermentation of Sclerotium sp., or Rhizobium sp., xanthan gum, and xanthan gum modified with glucose/mannose/glucuronic acid groups.

Thickening agents may also be synthetic. Such synthetic thickeners are typically based on polyacrylates although polyacrylamides can be equally used, such as acrylate copolymers and/or acrylate-alkyl acrylate copolymers. Also suitable are copolymers of C10.30-alkyl acrylates and one or more monomers of acrylic acid, of methacrylic acid or esters thereof which are crosslinked with an alkyl ether of sucrose or an alkyl ether of pentaerythritol. Other examples are compounds with INCI name "acrylates/C10.30 alkyl acrylate crosspolymer", Compounds with INCI name "acrylates/c12-24 pareth-25 acrylate copolymer, INCI name "acrylates/steareth-20 methacrylate copolymer", INCI name "acrylates/steareth-20 itaconate copolymer", INCI name "acrylates/aminoacrylates/C10.30 alkyl PEG-20 itaconate copolymer" and similar polymers

Mineral thickeners can be equally used. These are naturally occurring, mined ingredients that can absorb water or oils and boost viscosity. They give a different kind of viscosity than the natural gums. Materials include silica, bentonite, and magnesium aluminum silicate. These thickeners can be used to thicken oils as well as water-based formulations.

According to embodiments of the invention, the hydrophilic phase further comprises a thickening agent to prevent creaming, which thickening agent may be a thickening agent of plant origin, a synthetic agent or a mineral thickener.

An aspect according to the invention relates to the use of a cosmetic composition comprising a combination of at least one saponin as described herein and at least one saccharide polymer selected from the group consisting of starch hydrolysates, fructans, fructooligosaccharides, and mixtures thereof, in the preparation of a rinse-off cosmetic product or a leave-on cosmetic product.

According to embodiments of the invention, the rinse-off cosmetic product may be a rinse-off skin care product or a rinse-off hair care product, including hair conditioner, shampoo, shower gel, liquid soap gel, shaving cream, and the like.

According to embodiments of the invention, the leave-on cosmetic product may be a leave-on skin care product or a leave-on hair care product, including a styling hair gel, leave-on conditioner, facial cream, body lotion, lotions, a sun blocker cream, and the like.

The resulting rinse-off cosmetic products or leave-on cosmetic products are also aspects of the present invention. The use of the cosmetic composition described herein as a rinse-off cosmetic product or a leave-on cosmetic product is also an aspect of the invention.

## EXAMPLES

The present invention is further illustrated by the examples in the Experimental Section below.

Referring to the shampoo compositions as described in **Example 1** and the assessment of said compositions as described in **Example 2**, the following observations were made.

The addition of inulin (e.g. in CE-B) to a basic shampoo composition (represented in CE-A) seems to positively affect properties such as: *wet combing, wet detangling* and *volume,* and also had a less-pronounced effect on other parameters such as *dry combing, dry hair feel, shine* and *hair manageability.* Some of these effects are known from the state of the art and have been previously described in e.g. European patent application publication no. EP 1 380 284 A1. It can however also be seen that some other properties were not affected by the presence of inulin. Comparative examples CE-C and CE-D further show that such effects pertain even if the surfactant composition is slightly altered.

Furthermore, the partial and minor replacement in a basic type of shampoo of either a non-ionic surfactant (as in CE-E), an anionic surfactant (as in CE-F) or an amphoteric surfactant (as in CE-G) by saponin, the saponin being a non-ionic surfactant itself, did not have a profound influence on any of the parameters evaluated, apart from an apparent effect on dry combing and dry hair feel.

It was however found that the combination of saponin and inulin in a shampoo composition (as in E1-E3) yielded an additional unexpected effect in comparison with similar compositions CE-A to CE-G containing either inulin or saponin alone. When comparing the ratings accorded to the composition E1, with the ratings accorded to CE-A, CE-B and CE-E, it can be concluded that the former has a noticeable higher appreciation for parameters such as *wet combing, wet detangling* and *dry combing.*

Referring now to the shampoo compositions as described in **Example 3** and the assessment of said compositions as described in **Example 4**, the following observations were made.

The addition of inulin (i.e. CE-I) to a basic type of shampoo composition (CE-H) resulted in a more positive appreciation for the following parameters: *wet combing, wet detangling,* and *volume,* as well as a smaller but still noticeable effect for the following parameters: *dry combing, dry hair feel,* and *shine.* This observation is in line with the result reported here above.

Similar to Example 2, the addition of saponin did not have a noticeable effect on any of the tested parameters.

Furthermore, it was observed that the addition of both saponin and inulin to a basic type of shampoo composition resulted in more positive ratings for some of the tested parameters than could be expected on the basis of the ratings for the addition of inulin (i.e. CE-I) or saponin (i.e. CE-J) alone. Said parameters include *wet combing, wet detangling, dry combing,* and *hair manageability.*

Referring now to the shower gel compositions as described in **Example 5** and the assessment of said compositions as described in **Example 6**, the following observations were made.

The addition of inulin (e.g. CE-L) to a basic shower gel composition (as in CE-K) does not seem to have a significant influence on *dryness* and *smoothness,* but the conditioning *afterfeel* after 1 and 30 minutes is improved.

Rather unexpectedly, the addition of saponin (as in CE-M) had a positive effect on all the parameters that were tested, except for *distribution.*

The combined addition of saponin and inulin in i.e. E7-E9 were found to receive better ratings with respect to the parameter *afterfeel* after 1 and 30 minutes, than would be expected based on the ratings for shower gel compositions with only inulin (i.e. CE-L) or saponin (i.e. CE-M) added.

Referring now to the shower gel compositions as described in **Example 7** and the assessment of said compositions in **Example 8**, the following observations were made.

Similar to Example 6, the addition of inulin (i.e., CE-O) to a basic shower gel composition (i.e. CE-N) improves the *afterfeel* after 1 and 30 minutes.

The addition of saponin had a positive effect on all the parameters that were tested. This is in line with the results reported in Table 6.

The combined addition of saponin and inulin in shower gel compositions (as exemplified in E10-E12) were found to receive better ratings with respect to the parameter *afterfeel* after 1 and 30 minutes, than would be expected based on the ratings for shower gel compositions with only inulin (i.e. CE-O) or saponin (i.e. CE-P) added. However, this effect was less pronounced in comparison with Example 6.

Referring now to the cosmetic leave-on composition of **Example 9** and the assessment of said compositions in Example 10, the following observations were made.

The addition of inulin (as in e.g. CE-R) has a positive effect on the *skin feeling* afterwards, but also influences some other parameters negatively, such as *stickiness* and *skin penetration.* Regarding the positive effect on the *skin feeling,* it is remarkable that the *greasiness* feeling doesn't increase, while for typical care products not according to the invention *greasiness* tends to increase when the *skin feeling* gets richer.

The replacement of emulsifier GMS-SE by a small amount of saponin (as in e.g. CE-S), having however the same emulsifying effect, results in a considerable improvement for some parameters, including *spreadability, soaping effect, skin penetration, greasiness,* and *stickiness.* On the negative side, the *skin feeling* after use is less pronounced.

Advantageously, the combination of inulin and saponin has the unexpected advantage that the *skin feeling after use* can be improved with regard to a basic composition (as in CE-Q) or a composition with saponin only (CE-S), while keeping the advantages that are obtained by the addition of saponin, especially for *spreadability, stickiness,* and *soaping effect.* As such, the composition with inulin and saponin is experienced as "richer", meaning having a better afterfeel, while at the same time lacking the greasiness and stickiness which is commonly found and associated with rich creams with a good afterfeel.

## Saponin Extraction

Another aspect according to the invention relates to a process for preparing a cosmetic composition according to any of the preceding claims, the process comprising: extracting at least one saponin from its source using a mixture of water and a water-soluble alcohol; and combining said at least one saponin with at least one saccharide polymer selected from the group consisting of starch hydrolysates, fructans, fructooligosaccharides, and mixtures thereof. Aspects of the saponin extraction step will now be described in more detail. It should be noted that while the description is presented here as part of the process for preparing a cosmetic composition, these aspects form a novel and inventive method regardless of the subsequent use of the extracted saponin in the cosmetic compositions according to the invention.

The method pertains to extracting said at least one saponin from its plant source, by use of a mixture of water and a water-soluble alcohol.

It is known that saponins can be isolated from their plant source by extracting the saponin with water, usually demineralized water, a water-soluble alcohol or lower alcohol, or a combination of (demineralized) water and said alcohol. United States patent no. US 9,474,283, paragraphs [0132]-[0137] of which are incorporated herein by reference, describes a method for extracting saponin employing aqueous alcohol.

The presently disclosed method may comprise reducing the color intensity of a saponin extracted from a saponin source.

Typically, manufacturers of cosmetic products prefer as many of their basic ingredients to be as colorless as possible. However, most of the saponin sources or products which can be found on the market have a dark (e.g. brown) color. Even though only small amounts of saponin from these sources may be needed in a cosmetic composition, such small amounts may have a noticeable effect on the color of the final cosmetic product. It is therefore of importance to isolate the saponin from its source while reducing the color intensity, or color saturation, of the isolated product as much as possible.

Embodiments of the presently disclosed method comprise reducing the color intensity of a saponin extracted from a saponin source by said use of a mixture of water and a water-soluble alcohol, by using an alcohol:water mixture having a ratio of between 50:50 and 90:10, preferably between 55:45 and 85:15, more preferably between 60:40 and 80:20.

Embodiments of the presently disclosed method comprise reducing the color intensity of a saponin extracted from a saponin source by said use of a mixture of water and a water-soluble alcohol, by using of a combination of water and a lower alcohol for reducing the color intensity or color saturation of the saponin in the extraction process.

For the purpose of the invention, the term "lower alcohol" refers herein to a water-soluble alcohol, more in particular to an alcohol having no more than 7 carbon atoms. Preferably, said water-soluble alcohol is methanol, ethanol, 1-propanol, isopropyl alcohol or a butanol isomer.

It was now unexpectedly found that the color intensity of the extracted solution, which contains the major part of the saponin, can depend on the composition of the alcohol:water mixture. As such, a careful selection of a combination of water, preferably demineralized water, and a water-soluble alcohol, preferably methanol, ethanol, 1-propanol, isopropanol and mixtures thereof, allows reducing the color intensity, or color saturation, of the extract solution.

**Tables 17-19** show examples of extractions of saponin performed with alcohol:water mixtures with a different concentration. For each table, which show mixtures in a sequence with increasing alcohol concentration, a turning point can be found where the color intensity of the extract drops. Advantageously, the extraction yield is however not affected. It was found that starting from a certain alcohol: water ratio, a 10% increase in alcohol concentration resulted in at least a 10% drop in measured color intensity, often a 20%, 30% or even 40% reduction in color intensity.

It was thereby found that such an acceptable tradeoff between the decrease in color intensity and the extraction yield was often realized for an alcohol: water ratio which was situated between 60:40 and 80:20.

While the invention has been described hereinabove with reference to specific embodiments, this is done to illustrate and not to limit the invention, the scope of which is defined by the accompanying claims. The skilled person will readily appreciate that different combinations of features than those described herein are possible without departing from the scope of the claimed invention.

## EXPERIMENTAL SECTION

### General Procedure for carrying out the invention

As saccharide polymers are used:

-    INUTEC H25P: fructo-oligosaccharide from chicory (CreaChem)

- INUTEC HSI: fructo-oligosaccharide from chicory (CreaChem)
- INUTEC N10: native inulin from chicory (CreaChem)
- Orafti Bio: inulin from Agave (BENEO Orafti)

The general procedure for producing the cosmetic rinse-off compositions described herein included mixing with a propeller mixer at about 300 rpm the ingredients until a homogenous mixture was obtained.

The general procedure for producing the cosmetic leave-on compositions described herein included the following steps: water, preferably demineralized water, wherein xanthan gum has been predispersed, is mixed with all water-soluble ingredients and preservatives of the composition (including the at least one saponin) until a homogeneous mixture is obtained. Said aqueous mixture is then brought to a temperature of 70-75 °C. All remaining ingredients are mixed separately until a homogeneous mixture is obtained and brought to a temperature of 70-75 °C, and is then gradually added to the aqueous mixture under homogenisation using an Ultra-Turrax device at 10000 RPM, which continues for at least a further 3 minutes at 10000 RPM after completing the addition.

Products that were subjected to the evaluation by an independent test panel were always blind-coded and the order of the test presentation was fully randomized. The functionality of the cosmetic compositions as described in Examples 2, 4, 6, 8, and 10 were tested by a panel comprising 12 persons, the female:male ratio being 7:5. The persons that were part of said independent panel had no part in the making of or the research leading to the invention, nor were they linked in any way to the invention. With reference to the terms "blind" or "blind-coded" conditions, it is understood that none of the members of the test panel had any information whatsoever regarding the nature and identity of the test sample. Each member of the test panel conducted the test in absence of any other members. Each of the tested cosmetic compositions was evaluated on the basis of a selection of parameters, wherein each member of the test panel was asked for an appreciation by evaluating said parameters. Said evaluation was obtained by rating said parameters or sensory properties on a scale of 0-100, in increments of 5. It is thereby understood that a higher value represents a more positive (favorable) appreciation of the examined property. Said ratings were subsequently averaged. It is thereby to be understood that a difference of 10 points and more indicates a considerable change for said parameter.

Compositions as described in **Examples 11-16** were tested by directly comparing the performance of a composition according to the invention to a similar composition, typically excluding one or both of said at least one saccharide polymer and said at least one saponin.

The color intensity of an extract was obtained by use of the following procedure: A 1% aqueous solution of the original extract was made and adjusted to a pH of 6 with an amount as low as possible of HCl/NaOH. The solution was then filtered using a 0.45 $\mu$m filter to remove as much impurities as possible. The refractive index of the solution was measured. The appropriate amount of solution was then transferred to a quartz cuvette and put in a spectrophotometer. The absorbance of the solution at 420 nm was measured. Finally, the color intensity (in Icumsa) of each extract was determined by the following formula (ICUMSA Color method GS 2/3-10):

$$Color\ Intensity\ (Icumsa) = \frac{Measured\ Absorbance * 100000}{Length\ Cuvette * Measured\ Refractive\ index}$$

wherein said Measured Absorbance is a value between 0 and 3; Length Cuvette is expressed in cm and the Measured Refractive Index is a Number between 0 and 100.

**Example 1 - Shampoo rinse-off composition**

According to the general principles described hereabove, shampoo compositions E1-E3 according to the invention as well as comparative examples CE-A to CE-G were prepared. For this purpose, SLES (28 wt%) (Sodium Laureth Sulfate, Sasol), Cocamidopropyl Betain (30 wt%) (Sabo), Amidet N (100%) (PEG-4 Rapeseedamide, Kao Chemicals), Neovis SF (Acrylates Copolymer, Lubrizol), INUTEC N10 (Inulin, CreaChem), Saponin (mixture of 50% Tea saponin 90% purity and 50% Soapnut saponin 70% purity, Naturalin) were mixed in water in the amounts as indicated in Table 1. A homogenous viscous formulation was obtained. All prepared formulations were stable after 4 weeks storage at 50°C.

Table 1

| (wt%) | CE-A | CE-B | CE-C | CE-D | CE-E | CE-F | CE-G | E1 | E2 | E3 |
|---|---|---|---|---|---|---|---|---|---|---|
| H$_2$O | qs. | qs. | qs. | qs. | qs. | qs. | qs. | qs. | qs. | qs. |
| SLES (100%) | 10.7 | 10.7 | 9.7 | 10.7 | 10.7 | 9.7 | 10.7 | 10.7 | 9.7 | 10.7 |

(continued)

| (wt%) | CE-A | CE-B | CE-C | CE-D | CE-E | CE-F | CE-G | E1 | E2 | E3 |
|---|---|---|---|---|---|---|---|---|---|---|
| Cocamidopropyl Betain (100%) | 1,5 | 1,5 | 1,5 | 1,2 | 1,5 | 1,5 | 1,2 | 1,5 | 1,5 | 1,2 |
| Amidet N (100%) | 1 | 1 | 2 | 2 | 0 | 1 | 1 | 0 | 1 | 1 |
| Neovis SF | 5 | 5 | 5 | 5 | 5 | 5 | 5 | 5 | 5 | 5 |
| INUTEC N10 | 0 | 0.5 | 0.5 | 0.5 | 0 | 0 | 0 | 0.5 | 0.5 | 0.5 |
| Saponin | 0 | 0 | 0 | 0 | 1 | 1 | 1 | 1 | 1 | 1 |

## Example 2 - Shampoo assessment

The compositions of Example 1 were assessed with regard to the following parameters: Wet combing, Wet hair feel, Wet detangling, Dry combing, Dry Hair feel, Static hair properties, Shine, Volume and Hair manageability.

The panelists were instructed to wash their hair and subsequently give an appreciation for each of said parameters, more in particular by rating the sensory properties on a scale of 0-100, in increments of 5. The results are presented in Table 2.

Table 2

| | CE-A | CE-B | CE-C | CE-D | CE-E | CE-F | CE-G | E1 | E2 | E3 |
|---|---|---|---|---|---|---|---|---|---|---|
| Wet combing | 25 | 60 | 65 | 60 | 25 | 30 | 25 | 80 | 75 | 80 |
| Wet hair feel | 50 | 50 | 50 | 50 | 50 | 50 | 50 | 50 | 50 | 50 |
| Wet detangling | 25 | 60 | 65 | 60 | 25 | 25 | 30 | 75 | 70 | 75 |
| Dry combing | 50 | 60 | 65 | 65 | 60 | 55 | 60 | 70 | 75 | 75 |
| Dry hair feel | 50 | 60 | 60 | 65 | 55 | 60 | 60 | 65 | 65 | 65 |
| Static hair properties | 50 | 50 | 50 | 50 | 50 | 50 | 50 | 50 | 50 | 50 |
| Shine | 50 | 60 | 60 | 60 | 50 | 55 | 50 | 65 | 65 | 65 |
| Volume | 30 | 80 | 75 | 80 | 30 | 30 | 35 | 80 | 80 | 80 |
| Hair manageability | 60 | 70 | 75 | 70 | 60 | 65 | 60 | 70 | 75 | 75 |

## Example 3 - Shampoo rinse-off composition

According to the general principles described hereabove, shampoo compositions E4-E6 according to the invention as well as comparative examples CE-H to CE-J were prepared. As described here above SLES (28 wt%) (Sodium Laureth Sulfate, Sasol), Cocamidopropyl Betain (30 wt%) (Sabo), Amidet N (100%) (PEG-4 Rapeseedamide, Kao Chemicals), Neovis SF (Acrylates Copolymer, Lubrizol), INUTEC N10 (Inulin, CreaChem), Saponin (mixture of 50% Tea saponin 90% purity and 50% Soapnut saponin 70% purity, Naturalin) were mixed in water in the amounts as indicated in Table 3. A homogenous viscous formulation was obtained. All prepared formulations were stable after 4 weeks storage at 50°C.

Table 3

| (wt%) | CE-H | CE-I | CE-J | E4 | E5 | E6 |
|---|---|---|---|---|---|---|
| $H_2O$ | qs. | qs. | qs. | qs. | qs. | qs. |
| SLES (100%) | 5 | 5 | 5 | 5 | 4 | 5 |
| Cocamidopropyl Betain (100%) | 5.5 | 5.5 | 5.5 | 5.5 | 5.5 | 4.5 |
| Amidet N (100%) | 1 | 1 | 0 | 0 | 1 | 1 |
| Neovis SF | 5 | 5 | 5 | 5 | 5 | 5 |

(continued)

| (wt%) | CE-H | CE-I | CE-J | E4 | E5 | E6 |
|---|---|---|---|---|---|---|
| INUTEC N10 | 0 | 0.5 | 0 | 0.5 | 0.5 | 0.5 |
| Saponin | 0 | 0 | 1 | 1 | 1 | 1 |

## Example 4 - Shampoo assessment

The compositions of Example 3 were assessed for the same parameters as Example 2. The results are presented in Table 4.

Table 4

| | CE-H | CE-I | CE-J | E4 | E5 | E6 |
|---|---|---|---|---|---|---|
| Wet combing | 35 | 75 | 35 | 85 | 90 | 85 |
| Wet hair feel | 50 | 55 | 55 | 55 | 55 | 55 |
| Wet detangling | 30 | 70 | 30 | 80 | 85 | 80 |
| Dry combing | 60 | 70 | 65 | 85 | 90 | 85 |
| Dry hair feel | 70 | 80 | 75 | 85 | 80 | 85 |
| Static hair properties | 50 | 50 | 50 | 50 | 50 | 50 |
| Shine | 55 | 65 | 55 | 70 | 70 | 70 |
| Volume | 35 | 85 | 35 | 85 | 90 | 85 |
| Hair manageability | 65 | 70 | 65 | 85 | 80 | 80 |

## Example 5 - Shower gel rinse-off composition

According to the general principles described hereabove, shower gel rinse-off compositions E7-E9 according to the invention as well as comparative examples CE-K to CE-M were prepared. For this purpose, SLES (28 wt%) (Sodium Laureth Sulfate, Sasol), Cocamidopropyl Betain (Sabo), Amidet N (100 wt%) (PEG-4 Rapeseedamide, Kao Chemicals), NaCl (Sodium Chloride, Akzo Nobel), INUTEC N10 (Inulin, CreaChem), Saponin (mixture of 50% Tea saponin 90% purity and 50% Soapnut saponin 70% purity from Naturalin) were mixed in water in the amounts as indicated in Table 5. A homogenous viscous formulation was obtained. All prepared formulations were stable after 4 weeks storage at 50 °C.

Table 5

| (wt%) | CE-K | CE-L | CE-M | E7 | E8 | E9 |
|---|---|---|---|---|---|---|
| $H_2O$ | qs. | qs. | qs. | qs. | qs. | qs. |
| SLES (100%) | 10.7 | 10.7 | 10.7 | 10.7 | 9.7 | 10.7 |
| Cocamidopropyl Betain (100%) | 1,5 | 1,5 | 1,5 | 1,5 | 1,5 | 1,2 |
| Amidet N (100%) | 1 | 1 | 0 | 0 | 1 | 1 |
| NaCl | 0.5 | 0.5 | 0.5 | 0.5 | 0.5 | 0.5 |
| INUTEC N10 | 0 | 0.5 | 0 | 0.5 | 0.5 | 0.5 |
| Saponin | 0 | 0 | 1 | 1 | 1 | 1 |

## Example 6 - Shower gel assessment

The compositions of Example 5 were assessed on the basis of the following parameters: Distribution, Dryness, Smoothness, Feeling Afterwards after 1 minute and Feeling Afterwards after 30 minutes. The panelists were instructed to wash a defined part of their skin and subsequently give an appreciation for each of said parameters, more in particular by rating the sensory properties on a scale of 0-100, in increments of 5.

The results are presented in Table 6.

Table 6

|  | CE-K | CE-L | CE-M | E7 | E8 | E9 |
|---|---|---|---|---|---|---|
| Distribution | 50 | 50 | 55 | 50 | 50 | 50 |
| Dryness | 50 | 55 | 80 | 75 | 75 | 75 |
| Smoothness | 50 | 55 | 80 | 75 | 75 | 75 |
| Afterfeel 1 min | 60 | 70 | 75 | 85 | 85 | 85 |
| Afterfeel 30 min | 60 | 70 | 75 | 85 | 90 | 85 |

**Example 7** - **Shower gel rinse-off composition**

According to the general principles described hereabove, shower gel rinse-off compositions E10-E12 according to the invention as well as comparative examples CE-N to CE-P were prepared. For this purpose, SLES (28 wt%) (Sodium Laureth Sulfate, Sasol), Cocamidopropyl Betain (30 wt%)(Sabo), Amidet N (100 wt%) (PEG-4 Rapeseedamide, Kao Chemicals), NaCl (Sodium Chloride, Akzo Nobel), INUTEC N10 (Inulin, CreaChem), Saponin (mixture of 50% Tea saponin 90% purity and 50% Soapnut saponin 70% purity from Naturalin) were mixed in water in the amounts as indicated in Table 7. A homogenous viscous formulation was obtained. All prepared formulations were stable after 4 weeks storage at 50 °C.

Table 7

| (wt%) | CE-N | CE-0 | CE-P | E10 | E11 | E12 |
|---|---|---|---|---|---|---|
| $H_2O$ | qs. | qs. | qs. | qs. | qs. | qs. |
| SLES (100%) | 5 | 5 | 5 | 5 | 4 | 5 |
| Cocamidopropyl Betain (100%) | 5.5 | 5.5 | 5.5 | 5.5 | 5.5 | 4.5 |
| Amidet N (100%) | 1 | 1 | 0 | 0 | 1 | 1 |
| NaCl, | 0.5 | 0.5 | 0.5 | 0.5 | 0.5 | 0.5 |
| INUTEC N10 | 0 | 0.5 | 0 | 0.5 | 0.5 | 0.5 |
| Saponin | 0 | 0 | 1 | 1 | 1 | 1 |

**Example 8** - **Shower gel assessment**

The shower gel compositions as described in Example 7 were assessed by a panel as described here above, on the basis of the same parameters as in Example 6. The results are presented in Table 8.

Table 8

|  | CE-N | CE-O | CE-P | E10 | E11 | E12 |
|---|---|---|---|---|---|---|
| Distribution | 40 | 45 | 55 | 50 | 50 | 50 |
| Dryness | 45 | 45 | 75 | 70 | 75 | 75 |
| Smoothness | 50 | 50 | 80 | 75 | 75 | 75 |
| Afterfeel 1 min | 65 | 75 | 85 | 90 | 90 | 95 |
| Afterfeel 30 min | 70 | 80 | 80 | 85 | 85 | 90 |

**Example 9** - **cosmetic leave-on composition**

According to the general principles described hereabove, a cosmetic leave-on composition E13 according to the invention as well as comparative examples CE-Q to CE-S were prepared, according to Table 9. For this purpose, Xanthan Gum (Jungbunzlauer), Euxyl PE9010 (Phenoxyethanol, Ethylhexylglycerin, Schülke), Glycerine (Cremer), Caprylic/Capric Triglyceride (Sternchemie GmbH), Cetearyl alcohol (Sabo), GMS-SE (Glyceryl Stearate SE, Sabo), INUTEC N10 (Inulin, CreaChem), and Saponin (mixture of 50% Tea saponin 90% purity and 50% Soapnut saponin 70% purity from Naturalin) were mixed in water in the amounts as indicated in Table 9. A homogenous viscous emulsion was obtained. All prepared emulsions were stable against coalescence after 4 weeks storage at 50 °C.

Table 9

| (wt%) | CE-Q | CE-R | CE-S | E13 |
|---|---|---|---|---|
| $H_2O$ | qs. | qs. | qs. | qs. |
| Xanthan Gum | 0.6 | 0.6 | 0.6 | 0.6 |
| Euxyl PE9010 | 0.9 | 0.9 | 0.9 | 0.9 |
| Glycerine | 3 | 3 | 3 | 3 |
| Caprylic/ Capric Triglyceride | 15+ | 15 | 15 | 15 |
| Cetearyl alcohol | 2 | 2 | 2 | 2 |
| GMS-SE | 5 | 5 | 0 | 0 |
| INUTEC N10 | 0 | 1 | 0 | 1 |
| Saponin (Choisun 60%) | 0 | 0 | 0.2 | 0.2 |

**Example 10** - **cosmetic leave-on composition assessment**

The compositions as described in Example 9 were assessed by a panel as described here above. The parameters that were examined were the following: Spreadability, Soaping Effect, Penetration of the skin, Greasiness, Stickiness and Feeling afterwards (more in particular after 5 minutes). It is thereby noted that for the parameters Soaping Effect, Greasiness and Stickiness, a lower value for said parameter is more appreciated by the test person, as such effects are to be avoided for leave-on applications. The panelists were instructed to apply compositions to a defined part of their skin and subsequently give an appreciation for each of said parameters, more in particular by rating the sensory properties on a scale of 0-100, in increments of 5. The results are presented in Table 10.

Table 10

| | CE-Q | CE-R | CE-S | E13 |
|---|---|---|---|---|
| Spreadability | 60 | 60 | 80 | 80 |
| Soaping Effect | 40 | 40 | 20 | 20 |
| Penetration | 40 | 30 | 90 | 80 |
| Greasiness | 80 | 80 | 45 | 55 |
| Stickiness | 50 | 60 | 0 | 0 |
| Afterfeel | 70 | 85 | 50 | 80 |

**Example 11 - emulsions of cosmetic compositions - increasing presence of oil**

Table 11

| (wt%) | CE-T | E14 | CE-U | E15 | CE-V | E16 | CE-W | E17 | CE-X | E18 | CE-Y | E19 | CE-Z | E20 | CE-Z' | E21 |
|---|---|---|---|---|---|---|---|---|---|---|---|---|---|---|---|---|
| H$_2$O | qs. | qs. | qs. | qs. | qs. | qs. | qs. | qs. | qs. | qs. | qs. | qs. | qs. | qs. | qs. | qs. |
| Inulin (Orafti Bio) | - | 1 | - | 1 | - | 1 | - | 1 | - | 1 | - | 1 | - | 1 | - | 1 |
| xanthan gum | 0.6 | 0.6 | 0.6 | 0.6 | 0.6 | 0.6 | 0.6 | 0.6 | 0.6 | 0.6 | 0.6 | 0.6 | 0.6 | 0.6 | 0.6 | 0.6 |
| Ethylhexyl glycerin, Phenoxyethanol | 1 | 1 | 1 | 1 | 1 | 1 | 1 | 1 | 1 | 1 | 1 | 1 | 1 | 1 | 1 | 1 |
| Glycerin | 3 | 3 | 3 | 3 | 3 | 3 | 3 | 3 | 3 | 3 | 3 | 3 | 3 | 3 | 3 | 3 |
| Tea saponin 60% | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Cetearyl Alcohol C1618 | 2 | 2 | 2 | 2 | 2 | 2 | 2 | 2 | 2 | 2 | 2 | 2 | 2 | 2 | 2 | 2 |
| Caprylic/Capric Triglyceride | 10 | 10 | 20 | 20 | 30 | 30 | 40 | 40 | 50 | 50 | 60 | 60 | 70 | 70 | 90 | 90 |
| NaOH 10% pH:6-7 | qs. | qs. | qs. | qs. | qs. | qs. | qs. | qs. | qs. | qs. | qs. | qs. | qs. | qs. | qs. | qs. |

According to the general principles described hereabove, emulsions of cosmetic compositions E14-E20' according to the invention as well as comparative examples CE-T to CE-Z' were prepared. All prepared emulsions were stable against coalescence after 4 weeks storage at 50 °C.

Table 11 discloses said cosmetic compositions, each having a different concentration of caprylic triglyceride. For each concentration level of caprylic triglyceride, two variants were produced - one containing a mixture of saponin and inulin and one containing only saponin - which were subsequently evaluated by the test panel. It was considered that for every concentration level of caprylic triglyceride, the variant having both saponin and inulin had an improved skin after feel compared to the variant containing only saponin, while at the same time having similar appreciation levels for greasiness and penetration

**Example 12** - emulsions of cosmetic compositions - different types of oil

According to the general principles described hereabove, cosmetic compositions E21-1 to E21-44 according to the invention as well as comparative examples CE-AA-1 to CE-AA-44 were prepared, according to Table 12, wherein the parameter "oil" refers to one of the following:

(1) Macadamia oil; (2) Jojoba oil; (3) Sweet Almond oil; (4) Soybean oil; (5) Ethylhexyl Stearate; (6) Decyl Oleate; (7) Isoamyl laurate; (8) Cetearyl Ethylhexanoate; (9) Isodecyl Neopenanoate; (10) Octyldodecanol; (11) Cetyl palmitate; (12) Isostearyl Isostearate; (13) Isononyl Isononanoate; (14) C12-15 Alkyl Benzoate; (15) Isopropyl Palmitate; (16) Isopropyl Mirystate; (17) Cetearyl Isononanoate; (18) Pentaerythrityl Tetracaprylate/Tetracaprate; (19) Ethylhexyl Palmitate; (20) PEG-6 Caprylic/Capric glycerides; (21) Coco-Caprylate; (22) Triethyl Citrate; (23) Peg-7 Glyceryl Cocoate; (24) Dicaprylyl Ether; (25) Coco-Capryalte/Caprate; (26) Dimethicone M100; (27) Cyclopentasiloxane; (28) Cyclopentasiloxane, Dimethicone; (29) Cyclopentasiloxane, Dimethiconol; (30) Dimethicone, Cetearyl Dimethicone Crosspolymer; (31) Cyclopentasiloxane, Cyclohexasiloxane; (32) Cyclopentasiloxane, C30-45 Alkyl Cetearyl Dimethicone Crosspolymer; (33) Aqua, Amodimethicone, C11-15 Pareth-7, Laureth-9, Glycerin, Trideceth-12; (34) Mineral oil; (35) Polydecene; (36) Isododecane; (37) Isohexadecane; (38) Squalane; (39) Alkyl Benzoate, Polysilicone-15; (40) Alkyl Benzoate, Ethylhexyl Methoxycinnamate; (41) Alkyl Benzoate, Butyl Methoxydibenzoylmethane; (42) Octocrylene; (43) Benzophenone-3; (44) Ethylhexyl triazone

Table 12

| | CE-AA-(1-44) | E21-(1-44) |
|---|---|---|
| $H_2O$ | qs. | qs. |
| Inulin (INUTEC N10) | 0 | 1 |
| xanthan gum | 0.6 | 0.6 |
| Ethylhexylglycerin, Phenoxyethanol | 1 | 1 |
| Glycerin | 3 | 3 |
| Tea saponin 60% (Choisun) | 0.5 | 0.5 |
| Cetearyl Alcohol C1618 50/50 | 2 | 2 |
| Oil (1 to 44) | 20 | 20 |
| NaOH 10% pH:6-7 | qs. | qs. |

All compositions were tested to be stable against coalescence after 4 weeks storage at 50 °C.

For each type of oil, two variants were produced - one containing a combination of inulin and saponin and one containing only saponin - that were subsequently evaluated by the test panel.

It was considered that for every type of oil, the variant having both saponin and inulin had an improved skin afterfeel compared to the variant containing only saponin, while at the same time having similar appreciation levels for greasiness and penetration.

**Example 13 - emulsions of cosmetic compositions - different types of saponin**

Table 13

| (wt%) | CE-AB | E22 | CE-AC | E23 | CE-AD | E24 | CE-AE | E25 |
|---|---|---|---|---|---|---|---|---|
| $H_2O$ | qs. | qs. | qs. | qs. | qs. | qs. | qs. | qs. |
| Starch hydrolysate (Glucidex 12 (Maltodextrin DE 12, Roquette Frères) | - | 1.5 | - | 1.5 | - | 1.5 | - | 1.5 |
| xanthan gum | 0.6 | 0.6 | 0.6 | 0.6 | 0.6 | 0.6 | 0.6 | 0.6 |
| Ethylhexylglycerin, Phenoxyethanol | 1 | 1 | 1 | 1 | 1 | 1 | 1 | 1 |
| Glycerin | 3 | 3 | 3 | 3 | 3 | 3 | 3 | 3 |
| Cetearyl Alcohol C1618 50/50 | 2 | 2 | 2 | 2 | 2 | 2 | 2 | 2 |
| Caprylic/Capric Triglyceride | 60 | 60 | 60 | 60 | 60 | 60 | 60 | 60 |
| NaoOH 10% pH:6-7 | qs. | qs. | qs. | qs. | qs. | qs. | qs. | qs. |
| Quillaja Saponaria 67% (Desert King) | 0.5 | 0.5 | - | - | - | - | - | - |
| Gynostemma pentaphyllum 60% (Greenherb,Biological Technology) | - | - | 0.5 | 0.5- | - | - | - | - |
| Soapnut 70% (Naturalin) | - | - | - | - | 0.5 | 0.5 | - | - |
| Green tea (50%) (Vital-Chem, Zhuhai Corp.) | - | - | - | - | - | - | 0.5 | 0.5 |

According to the general principles described hereabove, emulsions of cosmetic compositions E22-E25 according to the invention as well as comparative examples CE-AB to CE-AE were prepared. All prepared emulsions were stable against coalescence after 4 weeks storage at 50°C.

Table 13 discloses said cosmetic compositions, each containing a saponin of different origin. For each of said saponin types, one variant containing the starch hydrolysate and one variant without the starch hydrolysate was produced. Said compositions were subsequently evaluated by the test panel. It was considered that for every of the four saponins tested herein, an improved skin after feel was obtained for the variant containing the starch hydrolysate as compared to the variant containing only saponin. For every saponin type, both variants with or without the starch hydrolysate were found to receive similar appreciation levels for greasiness and penetration.

**Example 14 - emulsions of cosmetic compositions - different types of emulsion breakers**

Table 14

| (wt%) | E26 | E27 | E28 | E29 | E30 | E31 | E32 | E33 | E34 | E35 | E36 | E37 |
|---|---|---|---|---|---|---|---|---|---|---|---|---|
| H2O | qs. | qs. | qs. | qs. | qs. | qs. | qs. | qs. | qs. | qs. | qs. | qs. |
| Inulin (INUTEC N10) | 0.5 | 0.5 | 0.5 | 0.5 | 0.5 | 0.5 | 0.5 | 0.5 | 0.5 | 0.5 | 0.5 | 0.5 |
| Hydroxyethyl cellulose | | | | | | | | 0.8 | | | | |
| Xanthan gum | 0.6 | 0.6 | 0.6 | 0.6 | 0.6 | 0.6 | 0.6 | | 0.6 | 0.6 | 0.6 | 0.6 |
| Ethylhexylglycerin, Phenoxyethanol | 1 | 1 | 1 | 1 | 1 | 1 | 1 | 1 | 1 | 1 | 1 | 1 |
| Glycerin | 3 | 3 | 3 | 3 | 3 | 3 | 3 | 3 | 3 | 3 | 3 | 3 |
| Tea Saponin 90% purity ( Plantnat) | 0.5 | 0.5 | 0.5 | 0.5 | 0.5 | 0.5 | 0.5 | 0.5 | 0.5 | 0.5 | 0.5 | 0.5 |
| Cetearyl alcohol | 2 | 2 | 2 | 2 | 2 | 2 | 2 | 2 | 2 | 2 | 2 | 2 |
| Caprylic/Capric Triglyceride | 20 | 20 | 20 | 20 | 20 | 20 | 20 | 20 | 20 | 20 | 20 | 20 |

(continued)

| (wt%) | E26 | E27 | E28 | E29 | E30 | E31 | E32 | E33 | E34 | E35 | E36 | E37 |
|---|---|---|---|---|---|---|---|---|---|---|---|---|
| MgSO4 | 5 | - | - | - | - | - | - | - | - | - | - | - |
| NaCl | - | 5 | - | - | - | - | - | - | - | - | - | - |
| Dihydroxy acetone | - | - | 4 | - | - | - | - | - | - | - | - | - |
| Potassium Thioglycolate | - | - | - | 5 | - | - | - | - | - | - | - | - |
| Glycolic acid 70% | - | - | - | - | 10 | - | - | - | - | - | - | - |
| Glyceryl caprylate | - | - | - | - | - | 1.5 | - | - | - | - | - | - |
| Caprylyl glycol | - | - | - | - | - | - | 1.5 | - | - | - | - | - |
| Cetrimonium chloride CTA25 | - | - | - | - | - | - | - | 4 | - | - | - | - |
| Aqua, Cocamidopropyl Betaine | - | - | - | - | - | - | - | - | 5 | - | - | - |
| Aqua, Sodium Cocoyl Glutamate, Sodium | - | - | - | - | - | - | - | - | - | 10 | - | - |
| Hydrogen peroxide (30%) | - | - | - | - | - | - | - | - | - | - | 30 | - |
| Ethanol | - | - | - | - | - | - | - | - | - | - | - | 10 |
| pH corrected with NaOH/citric acid to | 5.3 | 5.3 | 4.3 | 10.9 | 3.5 | 6 | 6.4 | 6.5 | 6 | 6 | 6.8 | 3.6 |

According to the general principles described hereabove, emulsions of cosmetic compositions E26-E37 according to the invention were prepared. Table 14 discloses said cosmetic compositions, each containing a different emulsion breaking agent.

Unexpectedly, all prepared emulsions proved stable against coalescence after 4 weeks storage at 50 °C. All prepared emulsions received a positive evaluation by the test panel with regard to greasiness, penetration, and skin feel.

**Example 15 - emulsions of cosmetic compositions - W/O/W**

Table 15

| (wt%) | | CE-AF | E38 |
|---|---|---|---|
| A | Aqua | 59 | 59 |
| A | Glycerin | 3 | 3 |
| A | Magnesium Sulfate | 1 | 1 |
| A | Phenoxyethanol, Ethylhexylglycerin | 0.5 | 0.5 |
| A | Polyglyceryl-3 Polyricinoleate, Polyglyceryl-3 Ricinoleate | 5 | 5 |
| | | | |
| B | Macadamia Integrifolia Seed Oil | 10.5 | 10.5 |
| B | Ethylhexyl Stearate | 10.5 | 10.5 |
| B | Isohexadecane | 10.5 | 10.5 |
| | | | |

(continued)

| (wt%) | | CE-AF | E38 |
|---|---|---|---|
| C | Aqua | 59.2 | 58.7 |
| C | Inulin (INUTEC H25P, CreaChem) | 0 | 0.5 |
| C | Glycerin | 3 | 3 |
| C | Tea saponin extract + Soapnut extract, obtained by the process of the invention (5%+95%) | 0.5 | 0.5 |
| C | Phenoxyethanol, Ethylhexylglycerin | 0.5 | 0.5 |
| C | Xanthan Gum | 0.6 | 0.6 |

The W/O/W emulsions, as disclosed in Table 15, were obtained by use of the following procedure: the ingredients listed under "A", "B" and "C" were mixed in three separate containers until homogeneous mixtures were obtained. The mixture with "A" ingredients was subsequently added to the "B" mixture, homogenized in a centrifuge during 5 minutes under 12000 RPM and further mixed for a period of 30 minutes.

36.2 g of the homogenised mixture was then mixed with "C" mixture at 60 °C, and further homogenized in a centrifuge for a period of 3 minutes at 10000 RPM. Both W/O/W emulsions were stable for coalescence for at least 4 weeks when stored at 50°C uninterruptedly.

E38 was evaluated more positively than CE-AF in terms of skin feel and greasiness and penetration.

**Example 16 - emulsions of cosmetic compositions - different types of surfactant**

Table 16

| (wt%) | CE-AG | E39 | CE-AH | CE-AI | CE-AJ | CE-AK |
|---|---|---|---|---|---|---|
| $H_2O$ | Q.S. | Q.S. | Q.S. | Q.S. | Q.S. | Q.S. |
| Inulin, INUTEC HSI | - | 0.4 | - | 0.4 | - | 0.4 |
| Xanthan gum | 0.6 | 0.6 | 0.6 | 0.6 | 0.6 | 0.6 |
| Ethylhexylglycerin, Phenoxyethanol | 1 | 1 | 1 | 1 | 1 | 1 |
| Glycerine | 3 | 3 | 3 | 3 | 3 | 3 |
| Soapnut Saponin extract (*obtained by the process of the invention*) | 0.5 | - | - | - | - | - |
| | - | 0.5 | - | - | - | - |
| Steareth-21 | - | - | 0.5 | - | - | - |
| | - | - | - | 0.5 | - | - |
| Glyceryl Stearate, PEG-100 Stearate | - | - | - | - | 0.5 | - |
| | - | - | - | - | - | 0.5 |
| Cetearyl Alcohol C1618 50/50 | 2 | 2 | 2 | 2 | 2 | 2 |
| Caprylic/Capric Triglyceride | 10 | 10 | 10 | 10 | 10 | 10 |
| Citric acid pH:6-7 | qs. | qs. | qs. | qs. | qs. | qs. |

According to the general principles described hereabove, emulsions of cosmetic composition E39 according to the invention as well as comparative examples CE-AG to CE-AK were prepared as presented in Table 16.

The emulsions prepared with the soapnut saponin extract proved stable for coalescence for at least 4 weeks when stored at 50°C. In strong contrast therewith, the emulsions prepared with Steareth-21 and (Glyceryl Stearate, PEG-100 Stearate) as surfactants, showed coalescence after 1 week at 40° C.

Emulsion E39 was found to be less greasy than CE-AG, while having a similar good "after feel" on the skin.

Compositions CE-AI and CE-AK were found to have a better "after feel" than CE-AH and CE-AJ respectively, but all four emulsions did not have the required stability.

**Example 17** - **Extraction of Saponins from *Sapindus Mukorossi* shells by Ethanol/Water mixtures**

100 grams of as such Sapindus Mukorossi (Vehgroshop) shells and 400 grams of 100% demineralized water or an ethanol/water mixture (50:50, 60:40, 70:30 and 75:25 ratios) were weighted.

The ethanol/water mixture was obtained by mixing the appropriate amounts of 96 wt% EtOH and 100% demineralized water. The demineralized water or EtOH/H2O mixture were preheated to 60 °C.

The finely ground Soapnut shells were added to the heated demineralized water or heated ethanol/water mixture and were stirred for 2 hours at 60°C using a 3-bladed propeller stirrer (950-1050 rpm). Alternatively, a magnetic stirrer (900-1080 rpm) could be used. The hot extract was filtered through a Whatman 1 filter (Qualitative 125 mm Ø * 100 circles).

After filtration, a colored solution was obtained. The EtOH in the solution was then removed via evaporation under vacuum. The color intensity of the extract after ethanol evaporation was determined according to the procedure described earlier.

Table 17 presents an overview of the color intensity measurements of the extracts and their yields.

Table 17

| Solvent system used | Color intensity extract | Extraction Yield |
|---|---|---|
| 100 % H2O | 14792 | 77,23 |
| 50% EtOH:50% H2O | 13679 | 72,31 |
| 60% EtOH:40% H2O | 13393 | 84 |
| 70% EtOH:30% H2O | 10283 | 78,9 |
| 75% EtOH:25% H2O | 8857 | 77,16 |
| 96% EtOH | 5437 | 48,81 |

**Example 18 - Extraction of Saponins from *Sapindus Mukorossi* shells by Alcohol/Water mixtures**

100 grams of as such *Sapindus Mukorossi* (National Organic's) shells and 400 grams of 100% demineralized water, an ethanol/water mixture (50:50, 60:40, 75:25 and 85:15 ratios) or a n-butanol/water mixture (60:40, 80:20) were weighted. The ethanol/water mixture was obtained by mixing the appropriate amounts of 96 wt% EtOH and 100% demineralized water. The n-butanol/water mixture was obtained by mixing the appropriate amounts of >99.5% n-butanol and 100% demineralized water. The demineralized water or alcohol/H2O mixtures were preheated to 60 °C. The same procedure as in Example 17 for extracting the saponins was followed.

Table 18 presents an overview of the color results of the extracts and their yields.

Table 18

| Solvent system used | Color intensity extract | Extraction Yield |
|---|---|---|
| 100 % H2O | 10490 | 72,5 |
| 50% EtOH:50% H2O | 10088 | 81,1 |
| 60% EtOH:40% H2O | 10300 | 70,9 |
| 75% EtOH:25% H2O | 5504 | 69,4 |
| 85% EtOH:15% H2O | 5182 | 65,1 |
| 60% n-BuOH:40% H2O | 10000 | 65,4 |
| 80% n-BuOH:20% H2O | 5138 | 55,6 |

**Example 19 - Extraction of Saponins from *Camellia Oleifera* by Alcohol/Water mixtures**

100 grams of 'as such' Camelia Oleifera seed meal and 400 grams of 100% demineralized water, an ethanol/H2O mixture (50:50, 60:40 and 75:25 ratios) or an isopropanol/H2O mixture (60:40 and 80:20 ratios) were weighted. The ethanol/water mixture was obtained by mixing the appropriate amounts of 96 wt% EtOH and 100% demineralized water. The isopropanol/water mixture was obtained by mixing the appropriate amounts of >99.5 wt% isopropanol and 100% demineralized water. The demineralized water or alcohol/H2O mixtures were preheated to 60°C.

The meal was added to the heated iPOH mixture and then it was stirred for 2 hours with magnetic stirring (900-1080 rpm). The hot extract was filtered through Whatman 1 (Qualitative 125 mm Ø *100 circles).

After filtration, a colored solution was obtained. The alcohol in the solution was then removed via evaporation under vacuum. The color intensity of the extract after ethanol evaporation was determined according to the procedure described earlier.

Table 19 presents an overview of the color intensity measurements of the extracts and their yields.

Table 19

| Solvent system used | Color intensity extract | Extraction Yield |
| --- | --- | --- |
| 100 % H2O | 89405 | 21.64 |
| 50% EtOH:50% H2O | 96833 | 27.36 |
| 60% EtOH:40% H2O | 86693 | 26.21 |
| 75% EtOH:25% H2O | 75500 | 24.8 |
| 80% EtOH:20% H2O | 66100 | 24.6 |
| 60% iPOH:40% H2O | 89732 | 29.8 |
| 80% iPOH:20% H2O | 72666 | 28.87 |

**Claims**

1. A cosmetic composition comprising:

   at least one saccharide polymer selected from the group consisting of starch hydrolysates, fructans, fructooligosaccharides, and mixtures thereof; and
   at least one saponin.

2. The cosmetic composition according to claim 1, wherein said at least one saccharide polymer comprises a levan-type polymer or an inulin-type polymer.

3. The cosmetic composition according to claim 1 or claim 2, wherein said at least one saccharide polymer is present in the composition in a concentration of at least 0.01 wt% to at most 8 wt%, by weight relative to the total weight of the composition.

4. The cosmetic composition according to any of the preceding claims, wherein said at least one saponin is present in the composition in a concentration of at least 0.001 wt% to at most 15 wt% by weight relative to the total weight of the composition.

5. The cosmetic composition according to any of the preceding claims, wherein said composition further comprises at least one of the following: an anionic surfactant, a cationic surfactant, an amphoteric surfactant, a non-ionic surfactant, wherein said non-ionic surfactant is exclusive of a saponin.

6. The cosmetic composition according to any of the preceding claims, further comprising at least one oil selected from jojoba oil, isoamyl laurate, and cyclopentasiloxane; said at least one oil being comprised in a hydrophobic phase of a stable emulsion.

7. The cosmetic composition according to any of the preceding claims, further comprising at least one emulsion breaking agent selected from the following:

   - an antimicrobial agent, such as a medium-chain length linear vicinal diol, glyceryl caprylate, or caprylyl glycol;
   - an electrolyte, such as MgSO4 or NaCl;
   - a cationic molecule, such as cationic surfactants such as cetrimonium chloride;
   - an oxidising agent, such as hydrogen peroxide in a concentration between 10 and 30% (v/v);
   - a sunless tanning agent, such as dihydroxy acetone (DHA);
   - a reducing agent, such as potassium thioglycolate;

- an alpha hydroxy acid, such as glycolic acid;
- an alpha and beta hydroxy acid, such as salicylic acid;
- a surfactant, such as cocamidopropyl betaine, sodium cocoyl glutamate, or sodium lauroyl glutamate;
- a perfume compound.

8. The cosmetic composition according to any of the preceding claims, wherein said composition is a leave-on cosmetic product or is part of a leave-on cosmetic product, wherein said at least one polysaccharide is present in the cosmetic product in a concentration of at least 0.01 wt% to at most 8 wt%, by weight relative to the total weight of the cosmetic product.

9. The cosmetic composition according to claim 8, wherein said at least one saponin is present in the cosmetic product in a concentration of at least 0.001 wt% to at most 5 wt% by weight relative to the total weight of the cosmetic product.

10. The cosmetic composition according to claim 8 or claim 9, wherein the weight ratio of said at least one saponin to the total of all surfactants in the composition, excluding said at least one saponin, is at least 1:100 and at most 100:1.

11. The cosmetic composition according to any of the preceding claims, wherein said composition is a rinse-off cosmetic product or is part of a rinse-off cosmetic product, wherein said at least one saccharide polymer is present in the cosmetic product in a proportion of at least 0.01 wt% to at most 8 wt%, by weight relative to the total weight of the cosmetic product.

12. The cosmetic composition according to claim 11, wherein said at least one saponin is present in the cosmetic product in a concentration of at least 0.1 wt% to at most 15 wt% by weight relative to the total weight of the cosmetic product.

13. The cosmetic composition according to claim 11 or claim 12, wherein the weight ratio of said at least one saponin to the total of all surfactants in the composition, excluding said at least one saponin, is at least 1:100 and at most 100:1.

14. Use of the cosmetic composition according to any of the preceding claims in the preparation of a rinse-off cosmetic product or a leave-on cosmetic product.

15. A process for producing the cosmetic composition according to any of claims 1-13, the process comprising extracting saponin from a source using a mixture of water and a watersoluble alcohol; and combining said saponin with at least one saccharide polymer selected from the group consisting of starch hydrolysates, fructans, fructooligosaccharides, and mixtures thereof.

16. The process according to claim 15, wherein an alcohol:water ratio is between 60:40 and 80:20.

Europäisches
Patentamt

European
Patent Office

Office européen
des brevets

# EUROPEAN SEARCH REPORT

**Application Number**

EP 20 15 0812

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 104 757 667 A (HU CAIZHONG) 8 July 2015 (2015-07-08) | 1-4, 8-13,15, 16 | INV. A61K8/60 A61K8/73 |
| Y | * paragraph [0001] * <br> * test groups 3, 4 6 * <br> * embodiments 1-3 * <br> * claims * | 1-16 | A61K8/97 A61K8/9717 A61K8/9789 A61Q5/02 A61Q5/12 |
| X | JP 2016 067248 A (NAGASE & CO LTD) 9 May 2016 (2016-05-09) | 1-5,7-14 | A61Q17/00 A61Q19/00 |
| Y | * paragraphs [0001], [0032], [0036], [0037]; claims; example 1 * | 1-16 | |
| X | WO 2016/153262 A2 (B&C BIOPHARM CO LTD [KR]) 29 September 2016 (2016-09-29) | 1,2,4, 15,16 | |
| Y | * first paragraph; page 1 * <br> * page 20, line 2 - line 19 * <br> * page 21, line 12 - line 22; claims * | 1-16 | |
| X | PL 229 133 B1 (SUPROBION SPOLKA Z OGRANICZONA ODPOWIEDZIALNOSCIA SPOLKA KOMANDYTOWA []) 29 June 2018 (2018-06-29) | 1 | **TECHNICAL FIELDS SEARCHED (IPC)** <br> A61K <br> A61Q |
| Y | * paragraphs [0002], [0018], [0022], [0023], [0028], [0037]; claims * | 1-16 | |
| X | CN 109 044 948 A (BAI JIN) 21 December 2018 (2018-12-21) | 1-5,7-14 | |
| Y | * "Technical field"; page 1; example 4 * | 1-16 | |

The present search report has been drawn up for all claims

| Place of search | Date of completion of the search | Examiner |
|---|---|---|
| Munich | 3 April 2020 | Heller, Dorothée |

## ANNEX TO THE EUROPEAN SEARCH REPORT
## ON EUROPEAN PATENT APPLICATION NO.

EP 20 15 0812

This annex lists the patent family members relating to the patent documents cited in the above-mentioned European search report.
The members are as contained in the European Patent Office EDP file on
The European Patent Office is in no way liable for these particulars which are merely given for the purpose of information.

03-04-2020

| Patent document cited in search report | | Publication date | Patent family member(s) | | Publication date |
|---|---|---|---|---|---|
| CN 104757667 | A | 08-07-2015 | NONE | | |
| JP 2016067248 | A | 09-05-2016 | JP<br>JP | 6106141 B2<br>2016067248 A | 29-03-2017<br>09-05-2016 |
| WO 2016153262 | A2 | 29-09-2016 | NONE | | |
| PL 229133 | B1 | 29-06-2018 | NONE | | |
| CN 109044948 | A | 21-12-2018 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9300067 A1
- EP 1380284 A1
- US 20070207105 A1
- US 9474283 B

**Non-patent literature cited in the description**

- **ANWAR et al.** *Appl Environ Microbiol.,* June 2008, vol. 74 (11), 3426-3433